# EUROPEAN PATENT APPLICATION

(11) **EP 2 412 724 A1**
(43) Date of publication of application: **01.02.2012**
(21) Application number: 10305836.8
(22) Date of filing: 29.07.2010
(51) Int. Cl.: C07K 14/47, C12N 5/0735, A61K 35/12, A61K 31/7088

(54) **Regulation of Glypican 4 activity to modulate the fate of stem cells and uses thereof**

(71) Applicant: Centre National de la Recherche Scientifique (C.N.R.S), 75016 Paris (FR); Université de la Méditerranée - Aix-Marseille II, 13007 Marseille (FR)
(72) Inventor: Dono, Rosanna, 13260 Cassis (FR); Fico, Annalisa, 13006 Marseille (FR); Maina, Flavio, 13260 Cassis (FR)
(74) Representative: Blot, Philippe Robert Emile

(57) **Abstract**

The present invention thus relates to stem cells or progenitor cells wherein the expression and/or activity of a member of the glypican family is reduced or abolished for use for the treatment of a pathological condition selected from the group consisting of a degenerative disease, a cardiac disorder, a metabolic disease and an injury; by stem cell-based therapy.

## Description

The present invention relates to methods for modulating the properties of self-renewal and differentiation of stem cells and progenitor cells enabling avoiding the teratoma risks associated with cell-based therapies. The present invention therefore also relates to novel stem cell-based therapies for treating human pathologies such as degenerative diseases, cardiac disorders, metabolic diseases and injuries.

Despite several progresses have been made in clinics to counteract symptoms of different human diseases, the identification of efficient and long-lasting therapies remains a hot topic. This is particularly relevant in the context of injuries and of neuronal, cardiac and muscle degenerative diseases. Current therapies for this type of diseases focus on the administration of pharmacological reagents that can alleviate patient symptoms. However, these treatments often induce side effects and do not stop the disease progression. For example, patients suffering from Parkinson's disease are nowadays treated with L-dopa and dopamine receptor agonists. However, the effectiveness of such treatments tends to decrease after several years and induce side effects such as dizziness and hallucination.

Another major social and economical problem is linked to the treatment of cardiovascular diseases. Angiograms, bypass surgery and angioplasty represent a big business. Over one million heart angiograms are indeed performed each year, which represents a huge annual cost. Nevertheless, this expensive surgery is physically invasive and traumatic for heart patients. Additionally, based upon extensive analysis, it appears that most of this money is wasted. Indeed, upon evaluation of case histories, it has been shown that this kind of surgery is five to ten times more deadly than the disease, and, in many instances, it is not effective.

Accordingly, alternative and effective strategies are needed to counteract a number of devastating human diseases.

Research undertaken on stem cells has led to the possibility of using them to achieve such objectives. Stem cells, defined by their capacities for self-renewal and for differentiation into multiple cell types, hold great promise for applications in medicine and pharmacology. To date, they can be obtained by different means. For example, stem cells can be isolated from embryos (embryonic stem cells or ESCs; Martin et al. (1981) Proc. Natl. Acad. Science USA 78:7634-7638). They are also present in adult tissues, but their differentiation potential is more restricted than the ESCs which have the unique feature to differentiate into all body cell types. A new type of pluripotent stem cells has also been generated recently through the de-differentiation of normal skin cells. These stem cells are called induced pluripotent stem cells (iPS cells; Takahashi et al. (2007) Cell 131:861-872). They retain similar properties and unique therapeutic potential of ESCs, while enabling overcoming risks of immuno-rejection in transplants and ethical issues linked to embryo disruption.

Strategies presently under development include transplants of stem cells, the manipulation of patient's own stem cells and the use of scaffold materials that emit biochemical signals or drugs to spur stem cells into action. However, the application of stem cells in clinics still depends on the capability of researchers to overcome major practical challenges. First, isolation and maintenance of stem cells requires further improvement of culture conditions. Second, the efficiency of stem cell differentiation needs to be substantially enhanced. Third, differentiation of stem cells results into heterogeneous rather than homogeneous cell populations, a situation that interferes with drug discovery and therapy (Xi and Zhang (2008) J. Cell. Biochem. 105:1153-1160; Murry and Keller (2008) Cell 132:661-80). Fourth, these heterogeneous cultures contain remaining pluripotent stem cells that are prone to develop into tumours or teratomas when implanted *in vivo* (Blum et al. (2009) Cell Cycle 8:3822-30). Therefore, a prerequisite for the successful exploitation of stem cells in medicine and pharmacology is the establishment of effective and user-friendly methodologies acting on key regulators of stem cell biology, easily accessible and with pleiotropic functions.

Cell fate and behaviour are dependent on their micro-environment including soluble growth factors, extracellular matrix interactions and cell-cell interactions. Soluble growth factors are among the predominant signalling players. Specifically, they act alone or in combination to promote cell fate in physiological and pathological conditions and they trigger different biological outputs on targeted cells according to time and strength of their receptor activation. Cell surface proteins, such as the heparin sulphate proteoglycans, Glypicans (Gpc), play a leading role in regulating interaction levels of secreted peptides with cells.

Glypican proteins consist of a core domain linked to the cell membrane by GPI-anchor and with attached glycosaminoglycan (GAG) chains. It has been proposed that Glypicans, through GAG chains, bind to a multitude of extracellular-matrix proteins such as chemokines, growth factors/morphogens (Filmus et al. (2008) Genome Biol. 9:224; Fico et al. (2007) Cell Mol Life Sci.)*.* Upon binding, Glypicans can either promote interaction of these extracellular signals with receptors or sequester them to down-regulate/block receptor activation, depending on ligand concentrations in the surrounding environment (Gutierrez et al. (2010) Mol. Cell. Biol. 30:1634-1649; Yan et al. (2009) Developmental Cell 17:470-481). Genetic and embryological studies demonstrate that Glypicans regulate cell signalling events during morphogenesis and adult physiology (Galli et al. (2003) Development 130:4919-4929; Luxardi et al. (2007) Biochem. Biophys. Res. Comm. 352:55-60).

The present invention is based on the discovery by the inventors that the Glypican 4 protein is expressed by different stem cell types and is required to establish and maintain stem cell properties of self-renewal and differentiation. Specifically, genetic studies of the biological function of Glypican 4 in mouse ESCs, performed by the inventors, revealed that Glypican 4 is required to establish proper balance of self-renewal versus differentiation. In particular, the inventors showed that loss of Glypican 4 activity in ESCs causes premature differentiation when cells are deprived of pluripotent signals or when they are exposed to differentiation signals. Additionally, by performing *in vivo* assays using *gpc4^{mut}* ESCs, the inventors demonstrated that these cells, in contrast to wild-types, do not develop teratomas when transplanted in nude mice, but they are still competent to develop in all embryonic tissues. Altogether, the inventors' findings showed that *gpc4^{mut}* cells are pluripotent but with the intrinsic ability to differentiate prematurely when placed in non permissive self-renewal conditions.

The present invention thus relates to stem cells or progenitor cells wherein the expression and/or activity of a member of the glypican family is reduced or abolished for use for the treatment by stem cell-based therapy of a pathological condition selected from the group consisting of a degenerative disease, a cardiac disorder, a metabolic disease and an injury.

In a second aspect, the present invention relates to an *in vitro* method for producing stem cells or progenitor cells displaying an early differentiation when exposed to environmental conditions triggering cell differentiation, comprising reducing or abolishing the expression and/or activity of a member of the glypican family in stem cells or progenitor cells; thereby yielding stem cells or progenitor cells displaying an early differentiation when exposed to environmental conditions triggering cell differentiation.

A third aspect of the present invention concerns an *in vitro* method for producing induced pluripotent stem cells (iPS cells) from somatic cells, comprising transiently increasing or inducing then transiently decreasing or reducing the expression and/or activity of a member of the glypican family in the somatic cells, thereby yielding induced pluripotent stem cells.

In a fourth aspect, the present invention relates to stem cells, progenitor cells or iPS cells obtainable by an *in vitro* method as defined above.

A fifth aspect of the invention concerns stem cells or progenitor cells obtainable by an *in vitro* method as defined above for use for the treatment by stem cell-based therapy of a pathological condition selected from the group consisting of a degenerative disease, a cardiac disorder, a metabolic disease and an injury.

A sixth aspect of the invention relates to an *in vitro* method for producing differentiated cells from stem cells or progenitor cells comprising the steps consisting in:
a) producing stem cells or progenitor cells displaying an early differentiation when exposed to environmental conditions triggering cell differentiation by the method defined above; and
b) exposing the stem cells or progenitor cells produced in step a) to environmental conditions triggering cell differentiation;
whereby differentiated cells are produced.

A seventh aspect of the present invention targets the use of stem cells, progenitor cells or iPS cells produced by the *in vitro* method as defined above for toxicological screenings.

In an eighth aspect, the invention concerns the use of a member of the glypican family for specifically purifying stem cells, progenitor cells, cancer stem cells or tissue specific stem cells from a sample.

A ninth aspect of the invention relates to an *in vitro* method for specifically purifying stem cells, progenitor cells, cancer stem cells or tissue specific stem cells from a sample, wherein the sample is contacted with a ligand of a member of the glypican family; thereby purifying stem cells, progenitor cells, cancer stem cells or tissue specific stem cells which are present in the sample.

A tenth aspect of the invention concerns a modulator of the activity of a member of the glypican family for use for the treatment of cancer.

An eleventh aspect of the invention relates to an antagonist of the activity of a member of the glypican family for use for inducing tissue regeneration after a degenerative disease.

### Detailed description of the invention

### Cells

As used herein, the terms "somatic cell" or "differentiated cell" are used indistinctively and refer to a non-germ cell of a subject, that displays specific structures, provides specific functions and wherein the potentialities in the expression of the genes are restricted.

As used herein, the term "stem cell" refers to a cell that can undergo self-renewal (*i.e.* progeny with the same differentiation potential) and also produces progeny cells that are more restricted in differentiation potential. Accordingly, a stem cell according to the invention possesses the two following properties:
(i) self-renewal *i.e.* the ability to go through numerous cycles of cell division while maintaining the undifferentiated state; and
(ii) potential *i.e.* the capacity to differentiate into specialized cell types.

In the context of the invention, stem cells encompass totipotent stem cells, pluripotent stem cells, multipotent stem cells and oligopotent stem cells.

The term "totipotent stem cells" refers to cells that can differentiate into embryonic and extraembryonic cell types. Such cells can construct a complete, viable organism. These cells are produced from the fusion of an egg and sperm cell. Cells produced by the first few divisions of the fertilized egg are also totipotent.

The term "pluripotent stem cells" refers to stem cells that have the potential to differentiate into any of the three germ layers: endoderm, mesoderm and ectoderm. Pluripotent stem cells can give rise to any fetal or adult cell type. However, a single cell or a conglomerate of pluripotent cells cannot develop into a fetal or adult animal because they lack the potential to organize into an embryo (Mitalipov and Wolf (2009) Adv. Biochem. Eng. Biotechnol 114:185-199).

The term "multipotent stem cells" refers to stem cells that can differentiate into a number of cells, but only those of a closely related family of cells.

The term "oligopotent stem cells" refers to stem cells that can differentiate into only a few cells, such as lymphoid or myeloid stem cells.

In the context of the present invention, stem cells encompass embryonic stem cells (ESCs); fetal stem cells including stem cells of the embryo proper and of extra-embryonic tissues such as amniotic fluid stem cells, umbilical cord blood stem cells, Wharton's jelly stem cells, amniotic membrane stem cells and placenta stem cells; adult stem cells, cancer stem cells and induced pluripotent stem cells. Preferably, stem cells according to the invention encompass fetal stem cells, adult stem cells, cancer stem cells and induced pluripotent stem cells. Still preferably, stem cells according to the invention encompass fetal stem cells, adult stem cells and induced pluripotent stem cells.

The term "embryonic stem cells" refers to pluripotent stem cells derived from the epiblast tissue of the inner cell mass of a blastocyst or earlier morula stage embryos. Human embryos reach the blastocyst stage four to five days post fertilization, at which time they consist of 50 to 150 cells. Embryonic stem cells may also be defined by the expression of several transcription factors, such as Oct-4, Nanog and Sox2, and cell surface proteins such as the glycolipids SSEA3 and SSEA4 and the keratin sulphate antigens Tra-1-60 and Tra-1-81.

Preferably, the term "stem cells" in accordance with the invention does not comprise stem cells from human embryos. Still preferably, the stem cells according to the invention are not directly derived from a human embryo. Still preferably, embryonic stem cells which have been derived from publicly available and previously established stem cell lines fall within the meaning of the term "stem cells" as used in the present invention.

In particular, human embryonic stem cells derived from one of the cell lines described in Table 1 fall within the meaning of the term "stem cells" as used in the present invention.

**Table 1: Human embryonic stem cell lines**

| **Line** | **Karyotype** | **Passage available** | **Country of origin** | **Origin** |
|---|---|---|---|---|
| SA01 | 46XY | 25 | Sweden | Cellartis AB |
| VUB01 | 46XY | 73 | Belgium | AZ-VUB Bruxel |
| HUES 24 | 46XY | 26 | USA | Harvard |
| H1 | 46XY, 20q11.21 | 26 | USA | Wicell research Institute |
| H9 | 46XX | 27 | USA | Wicell research Institute |
| WT3 | 46XY | 35 | UK | UKSCB |

The term "fetal stem cells" refers to stem cells derived either from the fetus proper or preferably from the extra-embryonic tissues emerging during gestation including umbilical cord blood, amniotic fluid, Wharton's jelly, the amniotic membrane and the placenta.

"Amniotic fluid stem cells" correspond to stem cells present in the amniotic fluid. They represent a heterogeneous population derived from all three germ layers.

"Amniotic membrane stem cells" correspond to stem cells originating from the inner layer (amniotic epithelial cells) or from the outer layer (amniotic membrane mesenchymal stromal cells or amnion-derived stem cells) of the amniotic membrane or amnion.

"Umbilical cord blood cells" correspond to stem cells originating from the umbilical cord blood and include in particular hematopoietic stem cells and mesenchymal stem cells.

"Wharton's jelly stem cells", also called "umbilical cord matrix stem cells", "umbilical cord perivascular cells" or "umbilical cord stroma cells" correspond to stem cells originating from the umbilical cord's outer region, known as Wharton's jelly. The Wharton's jelly is the mucoid connective tissue surrounding the two arteries and the single vein of the umbilical cord.

"Placenta stem cells" correspond to stem cells derived from placenta tissues and include chorionic mesenchymal stromal cells and chorionic trophoblastic cells.

As used herein, the terms "adult stem cells", "somatic stem cells" and "tissue specific stem cells" are used indifferently and refer to undifferentiated cells, found in a developed organism that has the ability to divide and create another cell like itself and also to divide and create a cell more differentiated than itself. Preferably, adult stem cells are multipotent stem cells. Adult stem cells have been isolated from a wide variety of adult tissues including blood, bone marrow, brain, olfactory epithelium, skin, pancreas, skeletal muscle, and cardiac muscle. Each of these stem cells can be characterized based on gene expression, factor responsiveness, and morphology in culture. In the context of the invention, adult stem cells encompass hematopoietic stem cells, mammary stem cells, mesenchymal stem cells, adipose-derived stem cells, endothelial stem cells, neural stem cells, cardiac stem cells, olfactory adult stem cells and neural crest stem cells.

"Hematopoietic stem cells" refer to stem cells found in the bone marrow and which give rise to all the blood cell types.

"Mammary stem cells" refer to stem cells derived from the mammary gland and which give rise to both the luminal and myoepithelial cell types of the gland.

"Mesenchymal stem cells" refer to stem cells of stromal origin and are derived in particular from adipose tissue, lung, bone marrow, blood and teeth.

"Adipose-derived stem cells" refer to stem cells found in fat tissues.

"Endothelial stem cells" refer to the third type of stem cells found in the bone marrow.

"Neural stem cells" refer to stem cells found in the nervous system.

"Cardiac stem cells" refer to stem cells found in the heart.

"Olfactory adult stem cells" refer to stem cells found in the olfactory mucosa present in the lining of the nose.

"Neural crest stem cells" refer to stem cells found in the hair follicles, gastrointestinal tract, sciatic nerve, cardiac outflow tract and spinal and sympathetic ganglia and which appear to represent a remnant of the stem cells of the embryonic neural crest. Neural crest stem cells may give rise to neurons, Schwann cells, myofibroblast, chondrocytes and melanocytes.

As used herein, the term "cancer stem cells" refer to cancer cells that possess characteristics associated with normal stem cells, specifically the ability to give rise to all cell types found in a particular cancer sample. Preferably, cancer stem cells persist in tumors as a distinct population and cause relapse, resistance and/or metastasis by giving rise to new tumors. Examples of cancer stem cells are well-known from the skilled person and include in particular leukaemic stem cells, brain cancer stem cells, breast cancer stem cells, colon cancer stem cells, ovary cancer stem cells, pancreas cancer stem cells and prostate cancer stem cells.

As used herein, the term "induced pluripotent stem cell" or "iPS cell" refers to a type of pluripotent stem cell artificially derived (e.g., induced by complete or partial reversal) from an undifferentiated cell *(e.g.* a non-pluripotent cell), typically an adult somatic cell such as an adult fibroblast.

As used herein, the term "progenitor cells" refers to cells that have a cellular phenotype that is more primitive (e.g., is at an earlier step along a developmental pathway or progression than is a fully differentiated cell) relative to a cell which it can give rise to by differentiation. Often, progenitor cells also have significant or very high proliferative potential. Progenitor cells can give rise to multiple distinct differentiated cell types or to a single differentiated cell type, depending on the developmental pathway and on the environment in which the cells develop and differentiate. Preferably, in the context of the invention, the progenitor cells are not directly derived from a human embryo.

### Glypicans

In the context of the invention, the term "glypican family" refers to a group of heparan sulphate proteoglycans that are bound to the outer surface of the plasma membrane by a glycosyl-phosphatidylinositol (GPI) anchor. There are six glypican family members in the human genome: Glypican 1, Glypican 2, Glypican 3 also called intestinal protein OCI-5 or GTR2-2 or MXR7, Glypican 4 also called K-Glypican, Glypican 5 and Glypican 6. Glypican proteins are between 555 and 580 amino acids in length. They are in particular described in Filmus et al. (2008) Genome Biology 9:224.

All members of the glypican family carry an N-terminal secretory signal peptide, a putative hydrophobic C-terminal signal sequence needed for the attachment of the GPI anchor and two to four consensus sites for the insertion of glycosaminoglycan chains near the point of membrane attachment (Song and Filmus (2002) Biochim. Biophys. Acta 1573:241-246). They all display a significant conservation of their primary polypeptide sequences. In particular, 14 cysteine residues are conserved which are believed to form intramolecular disulfide bonds, giving all glypicans a conserved three-dimensional structure.

Typically, the amino acid sequence of human Glypican 1 is referenced under Swiss Prot Number P35052; the amino acid sequence of human Glypican 2 is referenced under Swiss Prot Number Q8N158; the amino acid sequence of human Glypican 3 is referenced under Swiss Prot Number P51654; the amino acid sequence of human Glypican 4 is referenced under Swiss Prot Number 075487; the amino acid sequence of human Glypican 5 is referenced under Swiss Prot Number P78333; and the amino acid sequence of human Glypican 6 is referenced under Swiss Prot Number Q9Y625. In the context of the invention, the above cited Swiss Prot references are those that were available on May 5, 2010.

Glypicans are predominantly expressed during morphogenesis of different embryonic structures (Song and Filmus (2002) Biochem. Biophys. Acta 1573:241-246). Because of their cellular localization, they are exposed to all cues present in the extracellular environment, in particular to secreted signal peptides, which are key regulators of cell biological processes underlying embryonic development, tissue homeostasis and pathophysiology. Glypicans have evolved as essential cell membrane modulators of these instructive signals. In particular, they ensure that these signaling cues are perceived at the cell membrane with the right strength and at the right time so that targeted cells can undergo towards defined cellular and developmental events (Galli et al. (2003) Development 130:4919-4929; Fico et al. (2007) Cell Mol. Life Sci.*;* Williams et al. (2010) Proc. Natl. Acad. Sci. 107:5869-5874; Capurro et al. (2008) Dev Cell. 14:700-11; Capurro et al. (2005) Cancer Res. 65:6245-54). In agreement with this, alterations of Glypican functions underlie human congenital malformations and cancer (Cano-Gauci et al. (1999) J Cell BioL 146(1):255-64; Capurro et al. (2005) Cancer Res. 65(14):6245-54).

The main function of Glypicans is to regulate the signaling of Wnts, Hedghogs (Hhs), fibroblast growth factors (FGFs), bone morphogenetic proteins (BMPs) and hepatocyte growth factor (HGF) (Topczewski et al. (2001) Developmental Cell 1:251-264; Song et al. (2005) J. Biol. Chem. 280:2116-2125; Yan and Lin (2007) Developmental Biology 312:203-216; Ohkawara et al. (2003) Development 130:2129-2138; Williamson (2007) Cancer Res. 67(1):57-65). Depending on the biological context, Glypicans can either stimulate or inhibit signaling activity.

Preferably, in the context of the invention the member of the glypican family is Glypican 4.

Glypican 4 is the prime Glypican gene expressed in mouse embryos by two signalling centres important for induction and patterning of the developing forebrain namely the anterior visceral endoderm cells and the anterior neural ridge cells. Expression of Glypican 4 also in the anterior neuroectodermal cells adjacent to the anterior neural ridge suggests that Glypican 4 may modulate signalling in both inducing and responding cells (Luxardi et al. (2007) Biochem. Biophys. Res. Comm. 352:55-60). During head-fold stages in mouse embryos, Glypican 4 is predominantly expressed in the presumptive forebrain territory. Subsequently, its expression persists in neuronal progenitors of the developing forebrain (Hagihara et al. (2000) Developmental Dynamics 219:353-367).

Typically, the amino acid sequence of Glypican 4 is as shown in SEQ ID NO: 1. Preferably, the member of the glypican family according to the invention is a protein comprising or consisting of a sequence that is at least 80% identical to SEQ ID NO: 1, more preferably a protein comprising or consisting of a sequence that is at least 85%, at least 90%, at least 95% or at least 99% identical to SEQ ID NO: 1.

As intended herein, a first amino acid sequence which is at least x% identical to a second amino acid sequence means that x% represents the number of amino acids of the first sequence which are identical to their paired amino acids of the second sequence when the two sequences are optimally aligned pairwise, with respect to the total length of the second peptide sequence. As intended herein, two sequences are said to be optimally aligned pairwise when x is maximal.

Alignment can be performed manually or automatically. Algorithms for automated aligning of peptide sequences are well-known to the man skilled in the art and are for example described in Altschul et al. (1997) Nucleic Acids Res. 25:3389 and implemented by softwares such as the Blast software. The "needle" program, which uses the Needleman-Wunsch global alignment algorithm (Needleman and Wunsch (1970) J. Mol. Biol. 48:443-453) to find the optimum alignment (including gaps) of two sequences when considering their entire length, may for example be used to calculate the percentage of identity in accordance with the invention. The needle program is for example available on the ebi.ac.uk world wide web site. The percentage of identity in accordance with the invention is preferably calculated using the EMBOSS::needle (global) program with a "Gap Open" parameter equal to 10.0, a "Gap Extend" parameter equal to 0.5, and a Blosum62 matrix.

Said amino acid sequence which is at least 80% identical to the sequence defined above may be obtained from the native peptide sequence by mutation, e.g. by insertion, deletion or substitution of at least one amino acid and/or by chemical treatment.

The member of the glypican family according to the invention may also consist of a fragment of the sequence SEQ ID NO: 1.

As used herein, the term "fragment" of a reference sequence refers to a sequence which has a length inferior to the one of the reference sequence. In the context of the present invention, the fragments may for example have a length comprised between 10 and 550 amino acids, more preferably between 50 and 450 amino acids, between 100 and 350 amino acids or between 150 and 250 amino acids.

Fragments according to the invention may be in particular fragments of a member of the glypican family and preferably include either the Glypican core protein devoid of the glycosaminoglycan (GAG) attachment sites or the N-terminal domain of the Glypican protein devoid of the glycosylphosphatidylinositol (GPI) anchor. These functional domains are for example described in Williams et al. (2010) Proc. Natl. Acad. Sci. USA. 107(13):5869-5874 and in Zitterman et al. (2010) Int. J. Cancer. 126(6):1291-1301.

### Stem cells or progenitor cells wherein the expression and/or activity of a member of the glypican family is reduced or abolished

A first aspect of the invention relates to the use of stems cells or progenitor cells, as defined above, wherein the expression and/or activity of a member of the glypican family is reduced or abolished.

As used herein, the term "expression" refers to the cellular processes involved in producing RNA and proteins and as appropriate, secreting proteins, including where applicable, but not limited to, for example, transcription, translation, folding, modification and processing.

As used herein, the term "activity of a member of the glypican family" includes any biological activity mediated by a member of the glypican family. For example, Glypicans regulate the signalling of Wnts, Hedgehogs, fibroblast growth factors (FGFs), bone morphogenetic proteins (BMPs) and hepatocyte growth factor (HGF).

Techniques to assess the activity of a member of the glypican family are well-known from the skilled person and are for example described in Williams et al. (2010) Proc. Natl. Acad. Sci. USA 107(13):5869-5874 and Yan et al. (2009) Developmental Cell 17:471-484. They include in particular assaying the activation of the signalling pathways triggered by Glypican ligands such as Wnt or FGFs. In particular, the activity of Glypican 4 may be assessed by assaying the activation of the Wnt pathway in ESCs or by assaying the activation of the FGF pathway in neural ectodermal cells.

The terms "decrease", "reduce", "abolish" or "inhibit" are all used herein generally to mean a decrease by a statistically significant amount. However, for avoidance of doubt, "reduce" or "decrease" or "inhibit" means a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (e.g. absent level as compared to a reference sample), or any decrease between 10-100% as compared to a reference level. Preferably, "abolish" means a decrease by at least about 90% or up to and including a 100% decrease (e.g. absent level as compared to a reference sample).

The term "statistically significant" or "significantly" refers to statistical significance and generally means a two standard deviation (2SD) below normal, or lower, concentration of the molecule. The term refers to statistical evidence that there is a difference. It is defined as the probability of making a decision to reject the null hypothesis when the null hypothesis is actually true. The decision is often made using the p-value.

The present inventors have shown that the reduction or abolishment of the expression and/or activity of a member of the glypican family in a stem cell or a progenitor cell enabled to induce an early differentiation of these cells when exposed to environmental conditions triggering cell differentiation. Accordingly, the stem cells or progenitor cells wherein the expression and/or activity of a member of the glypican family is reduced or abolished according to the invention are stem cells or progenitor cells displaying an early differentiation when exposed to environmental conditions triggering cell differentiation.

An object of the present invention is thus also an *in vitro* method for producing stem cells or progenitor cells displaying an early differentiation when exposed to environmental conditions triggering cell differentiation, comprising reducing or abolishing the expression and/or activity of a member of the glypican family in stem cells or progenitor cells; thereby yielding stem cells or progenitor cells displaying an early differentiation when exposed to environmental conditions triggering cell differentiation.

In the context of the invention, the term "differentiation" or "cell differentiation" refers to the process whereby a cell moves further down the developmental pathway and begins expressing markers and phenotypic characteristics known to be associated with a cell that is more specialized and closer to becoming a terminally differentiated cell. Differentiation is a developmental process whereby cells assume a more specialized phenotype, *e.g.,* acquire one or more characteristics or functions distinct from other cell types.

As used herein, the expression "cells displaying an early differentiation" refers to stems cells or progenitor cells that differentiate more quickly into a more specialized cell than wild-type or non-pretreated stem cells or progenitors cells when exposed to similar environmental conditions triggering cell differentiation. Typically, cells displaying an early differentiation according to the invention reach their next step of differentiation 1 to 5 days earlier, in particular 2 to 4 days earlier, preferably 3 days earlier than wild-type or non-pretreated cells when exposed to similar environmental conditions triggering cell differentiation.

As used herein, the expression "environmental conditions triggering cell differentiation" refers to culture conditions wherein stem cells or progenitor cells are cultured in order to trigger the differentiation of said cells into more specialized cells. Environmental conditions triggering cell differentiation encompass in particular the characteristics of the culture medium, the presence or absence of appropriate cell feeder layers, the conditions of temperature, humidity, CO₂ content of the atmosphere wherein the cells are cultured or transfection of cells with expressing transcription factors. As well known from the skilled person, environmental conditions triggering cell differentiation depend on the type of cultured cells and on the type of specialized cells to be produced. In particular, the differentiation towards a specific lineage may be achieved by activating endogenous transcription factors, transfecting the cells with ubiquitously expressing transfection factors, exposing the cells to selected growth factors or co-culturing the cells with cell types capable of lineage induction. Stem cells or progenitor cells may also be differentiated into the lineage of interest by a combination of growth factors and/or their antagonists. The primary inducers that may be added into the cell culture of stem cells or progenitor cells in order to obtain differentiated cells of specific tissue type are for example summarized in Trouson (2006) Endocrine Rev. 27:208-219. Preferably, the environmental conditions triggering cell differentiation according to the invention are environmental conditions triggering neural or cardiac differentiation.

Environmental conditions triggering neural differentiation are for example described in Fico et al. (2008) Stem Cells and Development 17:573-584, Ying et al. (2003) Nat Biotechnol. 21(2):183-186 and Vierbuchen et al. (2010) Nature. 463(7284):1035-1041. Typically, neural differentiation may be triggered by using the following protocol. At day 0, ESCs may be dissociated in a single-cell suspension and 1,000 cells/cm² may be plated on gelatin-coated plates. The culture medium is preferably replaced daily during differentiation process. Culture medium for neural differentiation (serum-free Knockout Serum Replacement (KSR)-supplemented medium) may typically contain knockout Dulbecco minimal essential medium supplemented with 15% KSR (Invitrogen), 2 mM glutamine, 100 U/ml penicillin/streptomycin, and 0.1 mM β-mercaptoethanol.

Environmental conditions triggering cardiac differentiation are for example described in Maltsev et al. (1993) Mech Dev. 44(1):41-50. Typically, cardiac differentiation may be triggered by using the following protocol. Embryonic bodies (EBs) may be prepared from ESCs in hanging drops (300 cells/30µl-drop) on the lid of 10 cm bacteriological dishes containing 5 ml PBS. Hanging drops containing EBs may be incubated at 37°C in an atmosphere containing 5% CO₂ for two days. The hanging drops may then be washed with DMEM medium supplemented with 20% FCS, re-seeded in a new dish and incubated at 37°C for another 3 days to generate 5 days old EBs. On day 5, EBs may be plated separately onto gelatin-coated 24-well plates for morphological analysis of beating cardiomyocytes or onto 100-mm tissue culture plates for RT-PCR and Western blot.

Techniques to reduce or abolish the expression and/or activity of a member of the glypican family in a stem cell or progenitor cell are well-known the skilled person and include for example the use of nucleic acid molecule inhibitors, and contacting the stem cells or progenitor cells with an antagonist of the member of the glypican family. Antagonists of members of the glypican family include in particular soluble Glypican 3 (described for example in Zitterman et al. (2010) Int J Cancer. 126(6):1291-301), Glypican 4 antibodies (described for example in Geraldes et al. (2007) Invest Ophthalmol Vis Sci 48:5750-5755), Glypican 3 antibodies (described for example in Ishiguro et al. (2008) Cancer Res. 68(23):9832-9838) and Glypican 5 antibodies.

Suitable nucleic acid molecule inhibitors include antisense RNAs or RNAi, such as short interfering RNAs (siRNA), short hairpin RNAs (shRNA), and microRNAs.

The use of antisense methods to inhibit the *in vivo* translation of genes is well known in the art (e.g., U.S. Patent No. 7,425,544; U.S. Patent No. 7,307,069; U.S. Patent No. 7,288,530; U.S. Patent No. 7,1791796). Antisense nucleic acids are nucleic acid molecules (e.g., molecules containing DNA nucleotides, RNA nucleotides, or modifications (e.g., modification that increase the stability of the molecule, such as 2'-O-alkyl (e.g., methyl) substituted nucleotides) or combinations thereof) that are complementary to, or that hybridize to, at least a portion of a specific nucleic acid molecule, such as an RNA molecule (e.g., an mRNA molecule) (Weintraub (1990) Scientific Am. 262:40-46). The antisense nucleic acids hybridize to corresponding nucleic acids, such as mRNAs, to form a double-stranded molecule, which interferes with translation of the mRNA, as the cell will not translate a double-stranded mRNA. Antisense nucleic acids used in the invention are typically at least 10-12 nucleotides in length, for example, at least 15, 20, 25, 50, 75, or 100 nucleotides in length. The antisense nucleic acid can also be as long as the target nucleic acid with which it is intended to form an inhibitory duplex. Antisense molecules directed to a member of the glypican family, in particular to Glypican **4**, which are known in the art, are particularly suited for use in the present invention. Such antisense molecules are for example described in Haupt et al. (2009) J Cell Physiol. 220(3):780-791; Taylor (2009) PLoS Pathog. 5(11):e1000666 and Umezu (2010) Cancer Sci. 101(1):143-148. Antisense nucleic acids can be introduced into cells as antisense oligonucleotides, or can be produced in a cell in which a nucleic acid encoding the antisense nucleic acid has been introduced, for example, using gene therapy methods.

siRNAs are double stranded synthetic RNA molecules approximately 20-25 nucleotides in length with short 2-3 nucleotide 3' overhangs on both ends. The double stranded siRNA molecule represents the sense and anti-sense strand of a portion of the target mRNA molecule, in this case a portion of the member of the glypican family, in particular of Glypican 4, mRNA sequence. siRNA molecules are typically designed to target a region of the mRNA target approximately 50-100 nucleotides downstream from the start codon. Upon introduction into a cell, the siRNA complex triggers the endogenous RNA interference (RNAi) pathway, resulting in the cleavage and degradation of the target mRNA molecule. In addition, various improvements of siRNA compositions, such as the incorporation of modified nucleosides or motifs into one or both strands of the siRNA molecule to enhance stability, specificity, and efficacy, have been described and are suitable for use in accordance with this aspect of the invention (see International application WO 2004/015107; International application WO 2003/070918; International application WO 1998/39352; U.S. Patent Application 2002/0068708; U.S. Patent Application 2002/0147332; U.S. Patent Application 2008/0119427).

Short or small hairpin RNA molecules are similar to siRNA molecules in function, but comprise longer RNA sequences that make a tight hairpin turn. Like siRNA, they silence gene expression via the cellular RNA interference pathway. shRNA is cleaved by cellular machinery into siRNA. Suitable shRNA molecules for inhibiting Glypican 4 expression that can be used in accordance with the methods of the present invention include, without limitation, shRNA molecules, commercialized by Sigma, of the following sequences:
SEQ ID NO: 2:
   CCGGGCCACTGGTTTAAGCAATGTTCTCGAGAACATTGCTTAAACCAGTGGCTTTTTG;
SEQ ID NO: 3:
   CCGGGCATCCCGTTACAAGAAGTTTCTCGAGAAACTTCTTGTAACGGGATGCTTTTTG;
SEQ ID NO: 4:
   CCGGGCAGAGAAATCCCTGAATGATCTCGAGATCATTCAGGGATTTCTCTGCTTTTTG;
SEQ ID NO: 5:
   CCGGCCTTCAGAGTTTGACTACAATCTCGAGATTGTAGTCAAACTCTGAAGGTTTTTTG;
SEQ ID NO: 6:
   CCGGGCCTGCAAAGTAAAGATGATTCTCGAGAATCATCTTTACTTTGCAGGCTTTTTTG;
SEQ ID NO: 7:
   CCGGGCAAATTGAAGCTCCAGGTTACTCGAGTAACCTGGAGCTTCAATTTGCTTTTTTG;
SEQ ID NO: 8:
   CCGGGCTATTATGAACATGCAGGATCTCGAGATCCTGCATGTTCATAATAGCTTTTTTG; and
SEQ ID NO: 9:
   CCGGGCTTCACGTTACAAGAAGTTTCTCGAGAAACTTCTTGTAACGTGAAGCTTTTTTG.

Considerations to be taken into account when designing an RNAi molecule include, *e.g.,* the sequence to be targeted, secondary structure of the RNA target and binding of RNA binding proteins. Methods of optimizing siRNA sequences will be evident to the skilled worker. Typical methods are described, *e.g.,* in Vickers et al. (2003) J Biol Chem 278:7108-71 18 and Yang et al. (2003) Proc Natl Acad Sci USA 99:9942-9947.

Methods of making nucleic acid inhibitors (e.g., siRNAs) are conventional and will be evident to the skilled worker. *In vitro* methods include, *e.g.,* processing the Glypican 4 sequence in a cell-free system (e.g., digesting long double-stranded RNAs with RNAse III or Dicer), transcribing recombinant double-stranded Glypican 4 DNA *in vitro,* and chemical synthesis of nucleotide sequences homologous to a Glypican 4 sequence (see, *e.g.,* Tuschl et al. (1999) Genes & Dev. 13:3191-3197). *In vivo* methods include, *e.g.,* (1) transfecting DNA vectors into a cell such that a substrate is converted into siRNA *in vivo* (see, *e.g.,* Kawasaki et al. (2003) Nucleic Acids Res 31:700-707), (2) expressing short hairpin RNAs from plasmid systems using RNA polymerase III (pol III) promoters (see, *e.g.,* Kawasaki et al. (2003) Nucleic Acids Res 31:700-707) and/or (3) expressing short RNA from tandem promoters (see, *e.g.,* Miyagishi et al. (2003) Nature Biotechnol 20:497-500). In general, to inhibit Glypican 4 expression in cells in culture, one or more siRNAs can be added to cells in culture media, typically to a final concentration of about 50-200 µg, preferably about 50 µg siRNA/ml. Any of a variety of conventional methods can be used to introduce siRNAs into cells, including transfection, electroporation, or other methods known in the art (see, *e.g.,* Hannon (2002) Nature 418:244-251). Nanoparticle methods such as those described by Schiffelers et al. (2004) Nucleic Acid Res. 32:e149 and fusion protein methods such as those described by Song et al. (2005) Nature Biotechnol 23:709-717 are also useful.

Commercially available kits, such as siRNA molecule synthesizing kits from PROMEGA^{®} (Madison, WI) or AMBION^{®} (Austin, TX) may be used to synthesize siRNA molecules. In other examples, siRNAs are obtained from commercial sources, such as from QIAGEN^{®} Inc. (Germantown, MD), INVITROGEN^{®} (Carlsbad, CA), AMBION (Austin, TX), DHARMACON^{®} (Lafayette, CO), SIGMA-ALDRICH^{®} (Saint Louis, MO) or OPENBIOSYSTEMS^{®} (Huntsville, AL).

As used herein, the expression "antagonist of the member of the glypican family" refers to a natural or synthetic compound that inhibits the induction of the signal mediated by the activation of the member of the glypican family, preferably by binding to the member of the glypican family. An antagonist of the member of the glypican family may be in particular a chemical compound, a carbohydrate that binds to the member of the glypican family, or an antibody specific of the member of the glypican family, preferably a neutralizing or blocking antibody specific of the member of the glypican family.

Neutralizing or blocking antibodies specific of a member of the glypican family, in particular specific of Glypican 4, are for example described in Ford-Perriss et al. (2003) Developmental Dynamics 227:170-184.

As used herein, the term "antibody" refers to immunoglobulin molecules and immunologically active portions of immunoglobulin molecules, *i.e.,* molecules that contain an antigen binding site that immunospecifically binds an antigen. As such, the term antibody encompasses not only whole antibody molecules, but also antibody fragments as well as variants (including derivatives) of antibodies and antibody fragments and unconventional antibodies. The term "antibody", as used herein, includes both monoclonal and polyclonal antibodies, and encompasses antibodies raised *in vivo, e.g.,* produced by an animal upon immunization by an antigen, and antibodies generated *in vitro, e. g.,* generated by hybridomas. The antibody according to the invention may be an antibody from a mammal, in particular an antibody from a mouse, a rat, a rabbit, a camelid or a human. The antibody according to the invention may also be an antibody from a shark.

Preferably, an antibody according to the invention is a monoclonal antibody. The term "monoclonal antibody" refers to an antibody of a single amino acid composition, that is directed against a specific antigen and that is produced by a single clone of B cells or hybridoma.

Methods for producing polyclonal and monoclonal antibodies that react specifically with an immunogen of interest are known to those of skill in the art (see, *e. g.,* Harlow and Lane (1989) Antibodies: A Laboratory Manual Cold Spring Harbor Press, NY; Stites et al. (eds.); Basic and Clinical Immunology (4th ed.) Lange Medical Publications, Los Altos, CA and Kohler and Milstein (1975) Nature 256:495-497). In order to produce antisera containing antibodies according to the invention with the desired specificity, the member of the glypican family of interest can be used to immunize suitable animals, *e.g.,* mice, rabbits, or primates. A standard adjuvant, such as Freund's adjuvant, can be used in accordance with a standard immunization protocol. The animal's immune response to the immunogen preparation may be monitored by taking test bleeds and determining the titer of reactivity to the antigen of interest. Further fractionation of the antisera to enrich antibodies specifically reactive to the antigen and purification of the antibodies can be accomplished subsequently, using methods well known from those skilled in the art.

Monoclonal antibodies may be obtained using various techniques familiar to those of skill in the art. Typically, spleen cells from an animal immunized with a desired antigen are immortalized, commonly by fusion with a myeloma cell (Kohler and Milstein (1976) Eur. J. Immunol. 6:511-519). Alternative methods of immortalization include, *e.g.,* transformation with Epstein Barr Virus, oncogenes, or retroviruses, or other methods well known in the art. Colonies arising from single immortalized cells are screened for production of antibodies of the desired specificity and affinity for the antigen, and the yield of the monoclonal antibodies produced by such cells may be enhanced by various techniques, including injection into the peritoneal cavity of a vertebrate host. Additionally, monoclonal antibodies may also be recombinantly produced upon identification of nucleic acid sequences encoding an antibody with desired specificity or a binding fragment of such antibody by screening a human B cell cDNA library according to the general protocol outlined by Huse et al. (1989) Science 246:1275-1281. A monoclonal antibody may also be produced using recombinant DNA methods (see, e.g., U.S Patent No. 4,816,567) or by phage-display. The term "phage display" refers herein to a method for selecting ligands expressed on a bacteriophage capsid and encoded by a nucleic sequence inserted in the capsid encoding gene. This method is well known from those skilled in the art and is especially described by Scott and Smith (1990) Science 249:386-390, and Marks et al. (1991) J. Mol. Biol. 222:581-597.

The term "antibody fragment" include, but is not limited to Fv, Fab, F(ab')₂, Fab', Fd, dAb, dsFv, scFv, sc(FV)₂, CDRs, nanobodies, diabodies, multi-specific antibodies formed from antibodies fragments and other antibody fragments that retain at least a portion of the variable region of an intact antibody. Antibody fragments may be produced by enzymatic or chemical cleavage of intact antibodies, such as with pepsin, papain or trypsin. Alternatively, they can be prepared using recombinant DNA techniques such as those described in US Patent Applications N° 513,957, 693,249, 789,619, 776,391, 799,770, 799,772 and 809,083, wherein antibody fragments are produced from the RNA of an antibody producing B-cell from an immunized animal using known recombinant techniques.

The term "Fab" denotes an antibody monovalent fragment having a molecular weight of about 50,000 Da and antigen binding activity, and consisting of the light chain variable domain (VL), the heavy chain variable domain (VH), the light chain constant domain (CL) and the first heavy chain constant domain (CH₁).

The "Fv" fragment is the N-terminal part of the Fab fragment and consists of the variable portions of one light chain and one heavy chain.

The term "F(ab')₂" refers to an antibody bivalent fragment having a molecular weight of about 100,000 Da and antigen binding activity, which comprises two Fab fragments linked by a disulfide bridge at the hinge region.

The term "Fab" refers to an antibody fragment having a molecular weight of about 50,000 Da and antigen binding activity, which is obtained by cutting a disulfide bond of the hinge region of the F(ab')₂ fragment.

The term "Fd" refers to an antibody fragment consisting of the VH and CH₁ domains.

The term "dAb" (Ward et al. (1989) Nature 341:544-546) refers to a single variable domain antibody, *i.e.* an antibody fragment which consists of a VH or VL domain.

A single chain Fv ("scFv") polypeptide is a covalently linked VH::VL heterodimer which is usually expressed from a gene fusion including VH and VL encoding genes linked by a peptide-encoding linker. "dsFv" is a VH::VL heterodimer stabilised by a disulfide bond. Divalent and multivalent antibody fragments can form either spontaneously by association of monovalent scFvs, or can be generated by coupling monovalent scFvs by a peptide linker, such as divalent sc(Fv)₂.

The term "diabodies" refers to small antibody fragments with two antigen-binding sites, which fragments comprise a VH domain connected to a VL domain in the same polypeptide chain (VH-VL). By using a linker that is too short to allow pairing between the two domains on the same chain, the domains are forced to pair with the complementarity domains of another chain and create two antigen-binding sites.

Unconventional antibodies include, but are not limited to, nanobodies, linear antibodies (Zapata et al. (1995) Protein Eng. 8:1057-1062), single domain antibodies, single chain antibodies, affybodies, oligobodies, avimers, minibodies and antibodies having multiple valencies (e.g., diabodies, tribodies, tetrabodies, and pentabodies). Nanobodies are the smallest fragments of naturally occurring heavy-chain antibodies that have evolved to be fully functional in the absence of a light chain. Nanobodies have the affinity and specificity of conventional antibodies although they are only half of the size of a single chain Fv fragment. The consequence of this unique structure, combined with their extreme stability and a high degree of homology with human antibody frameworks, is that nanobodies can bind therapeutic targets not accessible to conventional antibodies. Recombinant antibody fragments with multiple valencies such as multimeric scFvs (e.g., diabodies, tetrabodies) offer an improvement over the parent antibody since small molecules of 60-100 kDa in size provide faster blood clearance and rapid tissue uptake (See Power et al. (2003) Methods Mol Biol 207:335-350 and Wu et al. (1999) Tumor Targeting 4:47-58).

Various techniques for making unconventional antibodies have been described. Bispecific antibodies produced using leucine zippers are described by Kostelny et al. (1992) J. Immunol. 148:1547-1553. Diabody technology is described by Hollinger et al. (1993) Proc. Natl. Acad. Sci. USA 90:6444-6448. Another strategy for making bispecific antibody fragments by the use of single-chain Fv diners is described by Gruber et al. (1994) J. Immunol. 152:5368. Trispecific antibodies are described by Tutt et al. (1991) J. Immunol. 147:60.

"Affibodies" are small, simple proteins having a molecular weight of 6 kDa, composed of a three-helix bundle based on the scaffold of one of the IgG-binding domains of Protein A, a surface protein from the bacterium *Staphylococcus aureus.* This scaffold has excellent features as an affinity ligand and can be designed to bind with high affinity to any given target protein. The domain consists of 58 amino acids, 13 of which are randomized to generate Affibody^{®} libraries with a large number of ligand variants. Thus, the libraries consist of a multitude of protein ligands with an identical backbone and variable surface-binding properties.

"Avimers" are multimeric binding proteins or peptides engineered using *in vitro* exon shuffling and phage display. Multiple binding domains are linked, resulting in greater affinity and specificity compared to single epitope immunoglobin domains.

"Mini-antibodies" or "minibodies" are scFv polypeptide chains which include oligomerization domains at their C-termini, separated from the scFv by a hinge region (Pack et al. (1992) Biochem 31:1579-1584). The oligomerization domain comprises self-associating α-helices, *e.g.,* leucine zippers, which can be further stabilized by additional disulfide bonds. The oligomerization domain is designed to be compatible with vectorial folding across a membrane, a process thought to facilitate *in vivo* folding of the polypeptide into a functional binding protein. Minibodies may be produced using recombinant methods well known in the art (See, *e.g.* Pack et al. (1992) Biochem 31:1579-1584 and Cumber et al. (1992) J. Immunology 149B:120-126).

"Oligobodies" are synthetic antibodies. The specificity of these reagents has been demonstrated by Western blot analysis and immunohistochemistry. They have some desirable properties, namely that as their production is independent of the immune system, they can be prepared in a few days and there is no need for a purified target protein (Radrizzani et al. (1999) Medicina (B Aires) 59(6):753-8). Oligobodies are produced using recombinant methods well known in the art (Radrizzani et al. (2000) Medicina (B Aires) 60 Suppl 2:55-60).

A Fab fragment can be obtained by treating monoclonal antibodies according to the invention with a protease, papain. Also, such Fab fragment can be produced by inserting DNA encoding Fab of the above antibodies into a vector for prokaryotic expression system, or for eukaryotic expression system, and introducing the vector into a prokaryote or eukaryote (as appropriate) to express the Fab fragment.

A F(ab')₂ fragment can be obtained by treating monoclonal antibodies according to the invention with a protease, pepsin. Also, such F(ab')₂ fragment can be produced by binding Fab' fragments described below via a thioether bond or a disulfide bond.

A Fab' fragment can be obtained by treating F(ab')₂ fragment as defined above with a reducing agent, dithiothreitol, and optionally a blocking group for the sulfhydryl groups resulting in cleavage of disulfide linkages. Also, such Fab' fragment can be produced by inserting DNA encoding Fab' fragments of monoclonal antibodies according to the invention into an expression vector for prokaryote, or an expression vector for eukaryote, and introducing the vector into a prokaryote or eukaryote (as appropriate) to perform its expression.

These methods are described, for example, by Goldenberg, US. 4,036,945 and 4,331,647, Nisonoff et al. (1960) Arch. Biochem. Biophys. 89:230; Porter (1959) Biochem. J. 73:119; and Edelman et al. (1967) In: Methods in Enzymology vol 1, p422, Academic Press. Other methods of cleaving antibodies, such as separation of heavy chains to form monovalent light-heavy chain fragments, further cleavage of fragments, or other enzymatic, chemical or genetic techniques may also be used, so long as the fragments bind to a member of the glypican family as defined above.

A scFv can be prepared by constructing a structural gene comprising DNA sequences encoding the VH and VL domains connected by an oligonucleotide. The structural gene can then be inserted into an expression vector for prokaryote, or an expression vector for eukaryote, which is subsequently introduced into a prokaryote or eukaryote host cells (as appropriate) to express the scFv fragment. The recombinant host cells then synthesize a single polypeptide chain with a linker peptide bridging the two V domains. Such methods for producing scFvs are described, for example, in Whitlow et al. (1991) Methods : A Companion to Methods in Enzymology 2:97; Bird et al. (1988) Science 242:423-426; Ladner et al., U.S. 4,946,778; Pack et al. (1993) Biotechnology 11:1271-1277 and Sandhu (1992) Crit. Rev. Biotech. 12:437.

To generate a humanized scFv fragment, a well known technology called complementary determining region (CDR) grafting may be used, which involves selecting the CDRs from a donor scFv fragment, and grafting them onto a human scFv fragment framework of known three dimensional structure as described for example in W0 98/45322; WO 87/02671; US 5,859,205; US 5,585,089; US 4,816,567; and EP 0173494.

Another form of an antibody fragment is a peptide coding for a single CDR. CDR peptides can be obtained by constructing genes encoding the CDR of monoclonal antibodies according to the invention. Such genes are prepared, for example, by using the polymerase chain reaction to synthesize the variable region from RNA of antibody-producing cells, as described, for example, in Larrick et al. (1991) Methods: A Companion to Methods in Enzymology 2:106.

In a particular embodiment, an antibody according to the invention is a chimeric antibody.

A "chimeric" antibody refers to an antibody made up of components from at least two different sources. In certain embodiments, a chimeric antibody comprises a portion of an antibody derived from a first species fused to another molecule, *e.g.,* a portion of an antibody derived from a second species. In certain such embodiments, a chimeric antibody comprises a portion of an antibody derived from a non-human animal fused to a portion of an antibody derived from a human. In certain such embodiments, a chimeric antibody comprises all or a portion of a variable region of an antibody derived from a non-human animal fused to a constant region of an antibody derived from a human.

In particular, an antibody according to the invention may be a humanized antibody. A "humanized" antibody refers to a non-human antibody that has been modified so that it more closely matches (in amino acid sequence) a human antibody. In certain embodiments, amino acid residues outside of the antigen binding residues of the variable region of the non-human antibody are modified. For the most part, humanized antibodies are human immunoglobulins (recipient antibody) in which residues from a hypervariable region of the recipient are replaced by residues from a hypervariable region of a non-human species (donor antibody) such as mouse, rat, rabbit or non-human primate having the desired specificity, affinity, and capacity. In certain embodiments, a humanized antibody is constructed by replacing all or a portion of a CDR of a human antibody with all or a portion of a CDR from another antibody, such as a non-human antibody, having the desired antigen binding specificity. In certain embodiments, a humanized antibody comprises variable regions in which all or substantially all of the CDRs correspond to CDRs of a non-human antibody and all or substantially all of the framework regions (FRs) correspond to FRs of a human antibody. In certain such embodiments, a humanized antibody further comprises a constant region (Fc) of a human antibody. Furthermore, humanized antibodies may comprise residues that are not found in the recipient antibody or in the donor antibody. These modifications are made to further refine antibody performance.

The term "human antibody" refers to a monoclonal antibody that contains human antibody sequences and does not contain antibody sequences from a non-human animal. In certain embodiments, a human antibody may contain synthetic sequences not found in native antibodies. The term is not limited by the manner in which the antibodies are made. For example, in various embodiments, a human antibody may be made in a transgenic mouse, by phage display, by human B-lymphocytes, or by recombinant methods.

Preferably the antibody specific of the member of the glypican family according to the invention is a neutralizing or blocking antibody specific of the member of the glypican family.

As used herein, the term "neutralizing antibody" or "blocking antibody" refers to an antibody which is able to block or neutralize the biological activity of an antigen comprising the epitope to which the antibody specifically binds. According to the amount of neutralizing antibody used, a residual biological activity produced by the antigen may remain. A neutralizing antibody may block or reduce the biological activity *in vitro* and/or *in vivo.* In the context of the invention, a neutralizing antibody specific of a member of the glypican family refers to an antibody which binds to said member of the glypican family, thereby preventing and/or inhibiting the signalling mediated by said member of the glypican family.

Neutralizing or blocking antibodies specific of a member of the glypican family, in particular specific of Glypican 4, are for example described in Ford-Perriss et al. (2003) Developmental Dynamics 227:170-184.

Techniques to identify Glypican antagonists are well-known from the skilled person and typically include assaying the ability of candidate compounds to induce premature neural and cardiac differentiation in cells expressing Glypican protein.

Typically, expression of Glypican 4 may be reduced or abolished by using the following protocol. In a 10 cm tissue culture plate, cells may be seeded at around 10⁵ cells in 10 ml of complete media. Cells may be incubated at 37°C in a CO₂ incubator, preferably during 24 h, until they reach 70-80% confluent. Cells may then be transfected using Lipofectamine using standard procedures. For each transfection, 2 µg of shRNA may be combined with 500 ng of DNA plasmid containing a reporte gene (e.g. GFP). The expression of the reporter gene typically allows checking efficiency of cell transfection. After removing the transfection mixture, normal growth medium may be replaced and cells may be cultured for additional 24-48 h prior to analysis of Glypican 4 expression (e.g. semi-quantitative RT-PCR or quantitative RT-PCR).

Preferably, the self-renewal of the stem cells or progenitor cells produced by the above method is maintained as long as the produced stem cells or progenitor cells are not exposed to environmental conditions triggering cell differentiation as defined above. In particular, the stem cells or progenitor cells produced according to the invention do not differentiate spontaneously in prolonged cultures when they are not exposed to environmental conditions triggering cell differentiation as defined above.

Another object the invention also relates to the stem cells or progenitor cells obtainable or obtained by the *in vitro* method as defined above.

The stem cells or progenitor cells obtainable or obtained by the *in vitro* method as defined above may also be used to produce differentiated cells. The present invention therefore also relates to an *in vitro* method for producing differentiated cells from stem cells or progenitor cells comprising the steps consisting in:
a) producing stem cells or progenitor cells displaying an early differentiation when exposed to environmental conditions triggering cell differentiation by the method defined above; and
b) exposing the stem cells or progenitor cells produced in step a) to environmental conditions triggering cell differentiation as defined above;
whereby differentiated cells are produced.

### Method for producing induced pluripotent stem cells

Since the present inventors demonstrated that the loss of Glypican 4 function in stem cells caused premature and/or enhanced differentiation, they anticipated that the modulation of Glypican 4 activity in somatic cells could induce the dedifferentiation of these cells and therefore would enable producing induced pluripotent stem cells.

The present invention therefore also relates to an *in vitro* method for producing induced pluripotent stem cells (iPS cells) from somatic cells, comprising transiently increasing or inducing then transiently decreasing or reducing the expression and/or activity of a member of the glypican family in the somatic cells, thereby yielding induced pluripotent stem cells.

As used herein, the terms "increase", "enhance" or "activate" generally mean an increase by a statistically significant amount. For the avoidance of any doubt, the terms "increase" or "enhance" or "activate" mean an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

The terms "decrease" or "reduce" have herein the same meaning as the one defined in the above section *"Stem cells or progenitor cells wherein the expression and*/*or activity of a member of the glypican family is reduced or abolished".*

The term "statistically significant" or "significantly" refers to statistical significance and generally means a two standard deviation (2SD) above normal, or higher, concentration of the molecule. The term refers to statistical evidence that there is a difference. It is defined as the probability of making a decision to reject the null hypothesis when the null hypothesis is actually true. The decision is often made using the p-value.

As used herein, the expressions "transient increase/decrease of the expression and/or activity of the member of the glypican family" or "transiently increase/decrease the expression and/or activity of the member of the glypican family" mean that the expression and/or activity of the member of the glypican family is only increased/decreased during a determined period of time.

Techniques to transiently induce or increase the expression and/or the activity of a member of the glypican family in a somatic cell are well-known from the skilled person and include for example transiently transfecting the somatic cells with a nucleic acid coding for the member of the glypican family or transiently contacting the somatic cells with a member of the glypican family or an activator of a member of the glypican family.

In a preferred embodiment, the nucleic acid encodes a protein comprising or consisting of a sequence at least 80%, at least 85%, 90%, 95%, 99% or 100% identical to SEQ ID NO: 1.

As used herein, the term "transfection" or "transfecting" refers to the process of deliberately introducing nucleic acids into cells. As well-known from the skilled person, techniques of transfection include chemical-based transfection using cyclodextrin, polymers in particular cationic polymers such as DEAE-dextran or polyethylenimine, calcium phosphate buffer, liposomes, dendrimers or nanoparticles; electroporation, sonoporation, optical transfection; particule-based method such as gene gun, magnetofection or Magnet assisted transfection, impalefection; viral methods such as transduction; nucleofection or heat shock.

As used herein, the term "nucleic acid" refers to polynucleotides such as deoxyribonucleic acid (DNA), and, where appropriate, ribonucleic acid (RNA). The term should also be understood to include, as equivalents, analogs of either RNA or DNA made from nucleotide analogs.

As used herein, the expression "activator of the member of the glypican family" refers to a natural or synthetic compound that activates or enhances the induction of the signal mediated by the activation of the member of the glypican family. An activator of the member of the glypican family may be in particular a carbohydrate that binds to the member of the glypican family, or an activating antibody specific of the member of the glypican family.

Techniques to transiently reduce the expression and/or activity of a member of the glypican family in a cell are well-known the skilled person and include for example the use of nucleic acid molecule inhibitors, in particular transfection of the cells with a siRNA or a shRNA, and transiently contacting the stem cells or progenitor cells with an antagonist of the member of the glypican family, as defined in the above section *"Stem cells or progenitor cells wherein the expression and*/*or activity of a member of the glypican family is reduced or abolished".*

Without willing to be bound by a particular theory, it is believed that the transient increase of the member of the glypican family during a specific period of time is necessary to improve reprogramming efficiency and that the following transient decrease of the member of the glypican family is important to overcome teratoma risk.

Preferably, when the iPS cells produced by the above defined method are not exposed to environmental conditions triggering cell differentiation as defined above, their self-renewal is maintained.

The present invention also relates to the iPS cells obtainable or obtained by the *in vitro* method defined above.

### Uses of stem cells or progenitor cells

The present inventors demonstrated that the modulation of Glypican 4 in stem cells or progenitor cells enabled preventing tumour formation when injected *in vivo,* thus conferring a particular advantage for the application of the stem cells or progenitor cells wherein the expression and/or activity of Glypican 4 is reduced or abolished in regenerative medicine.

Another object of the present invention thus relates to stem cells or progenitor cells wherein the expression and/or activity of a member of the glypican family is reduced or abolished for use for the treatment by stem cell-based therapy of a pathological condition. In a preferred embodiment, the pathological condition is selected from the group consisting of a degenerative disease, a cardiac disorder, a metabolic disease and an injury.

The invention also relates to the stem cells or progenitor cells obtainable or obtained by the *in vitro* method defined above for use for the treatment by stem-cell based therapy of a pathological condition. In a preferred embodiment, the pathological condition is selected from the group consisting of a degenerative disease, a cardiac disorder, a metabolic disease and an injury.

The present invention also describes a method of treatment, by stem cell-based therapy, of a pathological condition in an individual, comprising administering to an individual in need thereof a therapeutically effective amount of stem cells or progenitor cells wherein the expression and/or activity of a member of the glypican family is reduced or abolished. In a preferred embodiment, the pathological condition is selected from the group consisting of a degenerative disease, a cardiac disorder, a metabolic disease and an injury.

The present invention also describes a method of treatment, by stem cell-based therapy, of a pathological condition in an individual, comprising administering to an individual in need thereof a therapeutically effective amount of stem cells or progenitor cells obtainable or obtained by the *in vitro* method defined above. In a preferred embodiment, the pathological condition is selected from the group consisting of a degenerative disease, a cardiac disorder, a metabolic disease and an injury.

In the context of the present invention, an "individual" denotes a human or non-human mammal, such as a rodent (rat, mouse, rabbit), a primate (chimpanzee), a feline (cat), a canine (dog). Preferably, the individual is human.

As used herein, the term "treatment" or "treating" refers to both therapeutic treatment and prophylactic or preventative measures, wherein the object is to prevent or to slow the development of the disease.

By a "therapeutically effective amount" is meant a sufficient amount of the cells to treat a disorder, at a reasonable benefit/risk ratio applicable to any medical treatment. It will be understood, that the total daily usage of the cells as disclosed herein will be decided by the attending physician within the scope of sound medical judgment. The exact amount required will vary depending on factors such as the type of disease being treated and the severity of the disorder.

In the context of the invention, a "degenerative disease" refers to a disease in which the function or structure of the affected tissues or organs progressively deteriorate over time, whether due to normal bodily wear or lifestyle choices such as exercise or eating habits. Examples of degenerative diseases include amyotrophic lateral sclerosis (ALS) or Lou Gehrig's disease, Alzheimer's disease, Parkinson's disease, multiple system atrophy, Niemann Pick disease, atherosclerosis, progressive supranuclear palsy, cancer, Tay-Sachs disease, diabetes, heart disease, keratoconus, inflammatory bowel disease (IBD), prostatitis, osteoarthritis, osteoporosis, rheumatoid arthritis and Huntington's disease. Preferably, the degenerative disease according to the invention is selected from the group consisting of amyotrophic lateral sclerosis, Parkinson's disease, multiple system atrophy, cancer, diabetes, heart disease and Huntington's disease.

In the context of the invention, the term "cardiac disorder" or "heart disease" refers to diseases affecting the heart. In particular cardiac disorders encompass coronary heart diseases, cardiomyopathies, cardiovascular diseases, ischaemic heart diseases, heart failures, hypertensive heart diseases, inflammatory heart diseases and valvular heart diseases.

"Coronary heart disease" refers to the failure of the coronary circulation to supply adequate circulation to cardiac muscle and surrounding tissue.

"Cardiomyopathy" refers to the deterioration of the function of the myocardium for any reason.

"Cardiovascular disease" refers to a disease that affects the heart itself and/or the blood vessel system, especially the veins and arteries leading to and from the heart.

"Ischaemic heart disease" refers to a disease characterized by ischaemia to the heart muscle, usually due to coronary artery disease.

"Heart failure" refers to a condition that can result from any structural or functional cardiac disorder that impairs the ability of the heart to fill with or pump a sufficient amount of blood throughout the body, therefore leading to the heart and body's failure.

"Hypertensive heart disease" refers to a heart disease caused by high blood pressure, especially localised high blood pressure.

"Inflammatory heart disease" refers to a disease involving the inflammation of the heart muscle and/or the tissue surrounding it.

"Valvular heart disease" refers to a disease process that affects one or more valves of the heart.

Preferably, the cardiac disorder according to the invention is selected from the group consisting of cardiomyopathy, ischaemic heart disease, heart failure, hypertensive heart disease and valvular heart disease.

In the context of the invention, a "metabolic disease" denotes a disease that disrupts normal metabolism. Preferably, the metabolic disease according to the invention is a carbohydrate metabolism disorder.

As used herein a "carbohydrate metabolism disorder" denotes a disease wherein the metabolism of carbohydrate, for example of glucose, is disrupted. Carbohydrate metabolism disorders include diabetes, high fasting glycemia, overweight and obesity.

As used herein, "diabetes" denotes a syndrome of disordered metabolism, usually due to a combination of hereditary and environmental causes, resulting in abnormally high blood sugar levels.

As used herein, "high fasting glycemia" denotes a syndrome of disordered metabolism, resulting in a glycemia of more than 6.1 mmol/l.

As used herein, "overweight" denotes a condition in which excess body fat has accumulated to such an extent that health may be negatively affected. Overweight is commonly defined as a body mass index of 25 kg/m² or higher.

As used herein, "obesity" denotes a condition in which excess body fat has accumulated to such an extent that health may be negatively affected. Obesity is commonly defined as a body mass index of 30 kg/m² or higher.

As used herein, the term "injury" refers to a damage or harm caused to the structure or function of the body caused by an outside agent or force, which may be physical or chemical, and either by accident or intentional. Injuries encompass in particular bruise, wounds, cuts, grazes, burns, fractures, joint dislocations, concussions, sprains and shocks.

"Bruise" is a hemorrhage under the skin caused by contusion. "Wounds", "cuts" and "grazes" refer to injuries to or through the skin, that cause bleeding. "Burns" are injuries caused by excess heat, chemical exposure, or cold. "Fractures" are injuries to bones. "Joint dislocation" is a displacement of a bone from its normal joint, such as a dislocated shoulder or finger. "Concussion" is mild traumatic brain injury caused by a blow, without any penetration into the skull or brain. "Sprain" is an injury which occurs to ligaments caused by a sudden over stretching. "Shock" is a serious medical condition where the tissues cannot obtain sufficient oxygen and nutrients.

As explained above, the present inventors demonstrated that the risk of teratoma development was overcome when stem cells wherein the expression of Glypican 4 was reduced were transplanted in the flank of nude mice, in contrast to wild-type cells.

Accordingly, in a preferred embodiment, when the stem cells or progenitor cells wherein the expression and/or activity of a member of the glypican family is reduced or abolished are used as defined above, the formation of teratomas is prevented.

In the context of the invention, a "teratoma" refers to an encapsulated tumor with tissue or organ components resembling normal derivatives of all three germ layers.

The invention also provides the use of stem cells, progenitor cells or iPS cells produced by the *in vitro* methods defined above for screening assays preferably *in vitro* screening assays, particularly pharmaceutical, genetic or toxicological screening assays, preferably for toxicological screenings.

### Modulator of the activity of a member of the glypican family and uses thereof

As specified above, cancer stem cells are cancer cells that have the property to be tumorigenic. Indeed, they may generate tumors through the stem cell processes of self-renewal and differentiation in multiple cell types. They are thought to persist in tumors and cause relapse and metastasis by giving rise to new tumors. Therefore, a strategy to treat cancer is to specifically target cancer stem cells.

The present inventors showed that the modulation of the expression and/or activity of Glypican 4 in stem cells enabled acting on the fate of these cells. In particular, reducing or abolishing the expression and/or activity of Glypican 4 in cancer stem cells may enable impairing the self-renewal of cancer stem cells and/or promoting expression of differentiation markers.

Therefore, another aspect of the present invention concerns a modulator of the activity of a member of the glypican family for use for the treatment of cancer.

The present invention also describes a method for treating cancer in an individual, comprising administering a therapeutically effective amount of a modulator of the activity of a member of the glypican family in an individual in need thereof.

In the context of the invention, the term "modulate" is used consistently with its use in the art, *e.g.,* meaning to cause or facilitate a qualitative or quantitative change, alteration, or modification in a process, pathway, or phenomenon of interest. Without limitation, such change may be an increase, decrease, or change in relative strength or activity of different components or branches of the process, pathway, or phenomenon.

As used herein, a "modulator" is an agent that causes or facilitates a qualitative or quantitative change, alteration, or modification in a process, pathway, or phenomenon of interest. In particular, a modulator of the activity of a member of the glypican family may be an activator of a member of the glypican family or an antagonist of a member of the glypican family. Preferably, the modulator of the activity of a member of the glypican family is an antagonist of the member of the glypican family.

As used herein, the expression "antagonist of the member of the glypican family" has the same meaning as the one defined in the above section *"Stem cells or progenitor cells wherein the expression and*/*or activity of a member of the glypican family is reduced or abolished".*

As used herein, the expression "activator of the member of the glypican family" has the same meaning as the one defined in the above section *"Method for producing induced pluripotent stem cells* ".

As used herein, the term "cancer" means and includes any malignancy, or malignant cell division or malignant tumour, or any condition comprising uncontrolled or inappropriate cell proliferation and includes without limitation any disease characterized by uncontrolled or inappropriate cell proliferation. Cancers include carcinomas, sarcomas, adenocarcinomas, adenomas, leukemias, lymphomas and myelomas. In particular a cancer may be or may include bladder cancer, breast cancer, colon and rectal cancer, endometrial cancer, kidney (renal cell) cancer, leukemia, lung cancer, melanoma, skin cancer (nonmelanoma), non-Hodgkin lymphoma, pancreatic cancer, prostate cancer, thyroid cancer, stomach cancer, liver cancer, ovarian cancer. A cancer may also be adrenal cancer, anal cancer, aplastic anemia, bile duct cancer, bladder cancer, bone cancer, brain cancer, breast cancer, bronchus and lung cancer, central nervous system cancer, cervical cancer, colon and rectum cancer, connective tissue cancer, cranial nerves cancer, digestive organs cancer, endometrial cancer, endocrine cancer, esophageal cancer, Ewing's family of tumors, gallbladder cancer, gastrointestinal carcinoid tumors, gastrointestinal stromal tumors, gestational trophoblastic disease, head and neck cancer, Hodgkin lymphoma cancer, Kaposi's sarcoma, kidney cancer, laryngeal and hypopharyngeal cancer, larynx cancer, leukaemia cancer, leukaemia - lymphoid cancer, leukaemia - myeloid cancer, leukaemia of unspecified cell type cancer, lip cancer, liver cancer, lung carcinoid tumors, lymphoid, haematopoietic and related tissues cancer, lymphoma cancer, malignant immunoproliferative cancer, malignant neoplasm cancer, malignant neoplasm of genital organs cancer, malignant neoplasm of urinary tract cancer, meninges cancer, mesothelioma cancer, multiple myeloma cancer, myelodysplastic syndrome, nasal cavity and middle ear cancer, nasopharyngeal cancer, nasopharynx cancer, neuroblastoma, non-Hodgkin's lymphoma cancer, non-Hodgkin's lymphoma, neuroblasma, oral cavity and oropharyngeal cancer, osteosarcoma, oropharynx cancer, ovarian cancer, pancreas cancer, parathyroid cancer, penis cancer, peripheral and cutaneous T-cell lymphomas cancer, peripheral nerves cancer, peritoneum cancer, pharyngeal cancer, pituitary tumor, postcricoid region cancer, prostate cancer, retinoblastoma, rhabdomyosarcoma, salivary glands cancer, sarcoma, soft tissue cancer, melanoma skin cancer, nonmelanoma skin cancer, sinus cancer, small intestine cancer, stomach cancer, testicular cancer, thymoma and thymic carcinoma, thyroid cancer, tongue cancer, tonsil cancer, trachea cancer, cancer NOS, urinary organs cancer, urethral cancer, uterine cancer, uterine sacrcoma, vaginal cancer, vulvar cancer, Waldenstrom macroglobulinemia, and Wilms tumour.

Preferably, the modulator of the activity of a member of the glypican family for use for the treatment of cancer as defined above impairs the self-renewal of cancer stem cells and/or triggers expression of differentiation markers (*e.g.* Piccirillo et al. (2006) Nature 444:761-765).

Another aspect of the present invention concerns the modulation of the activity of Glypican 4 in adult stem cells.

As specified above, adult stem cells or tissue specific stem cells are found in different tissues and possess the properties of self-renewal and differentiation. Accordingly, they are considered as an important therapeutical target for tissue regeneration after a disease. The present inventors propose in particular to act on adult stem cells *in loco,* by modulating the activity of Glypican 4, to enhance their differentiation capacity and promote tissue repair.

Accordingly, the present invention also relates to an antagonist of the activity of a member of the glypican family for use for inducing tissue regeneration, in particular after a degenerative disease.

The present invention also provides a method for inducing tissue regeneration in an individual, in particular after a degenerative disease, comprising administering a therapeutically effective amount of an antagonist of the activity of a member of the glypican family in an individual in need thereof.

As used herein, the expression "antagonist of the member of the glypican family" has the same meaning as the one defined in the above section *"Stem cells or progenitor cells wherein the expression and*/*or activity of a member of the glypican family is reduced or abolished".*

In the context of the invention, the terms "tissue regeneration" and "tissue repair" are interchangeably and refer to the proliferation, differentiation, survival and migration of cells capable of developing into the tissue to be repaired. In particular, in this pathway, functional cells of the particular tissue are regenerated, probably from tissue specific stem cells or tissue specific progenitor cells, resulting in regeneration of a healthy functional tissue. Accordingly, in the context of the present invention, the antagonist for use for inducing tissue regeneration is for enhancing differentiation of tissue specific stem cells, adult stem cells or tissue specific progenitor cells present in the tissue to be regenerated. Preferably, the antagonist is for use for enhancing differentiation of tissue specific stem cells present in the tissue to be regenerated.

Diseases or disorders that can be treated in this manner include in particular degenerative diseases as defined above, spinal cord injuries, cardiac diseases, stroke, autism, eye diseases, chronic obstructive pulmonary disease, autoimmune disorders, ischemic heart disease, cancer, wound healing and burns. Preferably, the disease that is treated in this manner is a degenerative disease as defined above.

Essentially any tissue or organ can be targeted for inducing tissue regeneration, including the brain, heart, vascular system, pulmonary system, renal system, splenic system, lymphatic system, bone marrow, bone, skeletomuscular system, immune system, reproductive system, skin, cartilage, nervous system, gastrointestinal system, liver, pancreatic system, hematopoietic system, a hormonal system, among others.

The modulators, antagonists and activators as defined above may be formulated under any suitable form, such as a suspension, a micro- or macro-emulsion, micelles, a depot formulation such as for depot injection, a dry formulation in particular suitable for topical and transdermal administration or in liposomes. They may also be formulated as a controlled release formulation, e.g. by combination with polymers such as PGA (polyglycolic acid) or with cyclodextrines.

Preferably, the modulators, antagonists and activators as defined above are administered in the form of pharmaceutical compositions further comprising at least one pharmaceutically acceptable carrier.

The term "pharmaceutically acceptable carrier" refers to a carrier that may be administered to a patient, together with a compound of this invention, and does not destroy the pharmacological activity thereof and is non-toxic when administered in doses sufficient to deliver a therapeutically effective amount of the compound.

Pharmaceutically acceptable carriers and vehicles that may be used in the compositions of this invention include, but are not limited to, ion exchangers, alumina, aluminium stearate, lecithin, self-emulsifying drug delivery systems (SEDDS) such as d-a-tocopherol polyethyleneglycol 1000 succinate, surfactants used in pharmaceutical dosage forms such as Tweens or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

As appreciated by skilled artisans, compositions are suitably formulated to be compatible with the intended route of administration. Examples of suitable routes of administration include parenteral, *e.g.,* intravenous, intradermal, subcutaneous, intramuscular, intraperitoneal, oral (e.g., buccal, inhalation, nasal and pulmonary spray), intradermal, transdermal (topical), transmucosal, intraocular, intratumoral and rectal administration.

The composition according to the invention may be delivered in doses ranging from about 0.01 µg/kg to about 5000 µg/kg, alternatively from about 0.1 to about 1000 µg/kg, alternatively from about 1 to about 500 µg/kg. Effective doses will also vary depending on route of administration, as well as the possibility of co-usage with other agents.

### Purification methods

Since Glypican 4 is predominantly present in different stem cell types, the present inventors proposed to use the presence of Glypican 4 on these cells to purify this type of cells from a mixture of cells that may include both stem cells and differentiated cells.

The invention therefore also relates to the use of ligand of a member of the glypican family for specifically purifying stem cells, progenitor cells, cancer stem cells or tissue specific stem cells from a sample.

The present invention also provides an *in vitro* method for specifically purifying stem cells, progenitor cells, cancer stem cells or tissue specific stem cells from a sample, wherein the sample is contacted with a ligand of a member of the glypican family; thereby purifying stem cells, progenitor cells, cancer stem cells or tissue specific stem cells which are present in the sample.

As used herein, a "sample" refers to a part of a bigger set. Preferably, a sample according to the invention is a substance of biological origin. Examples of biological samples include, but are not limited to, pieces of organs or of tissues such as kidney, liver, heart, lung, and the like, arteries veins and the like, blood and components thereof such as plasma, platelets, subpopulations of blood cells and the like.

In the context of the invention, the expression "specifically purifying stem cells" means isolating specifically stem cells among a mixture of components in particular among a mixture of cells. After specific purification, contaminants may still be present among stem cells but, preferably, stem cells represent the majority of the purified cells. For example, stem cells represent more than 85% of the purified cells, more preferably more than 90%, still preferably more than 95%, most preferably more than 99% of the purified cells.

As used herein, a "ligand of a member of the glypican family" refers to a molecule which has binding affinity towards a member of the glypican family. In particular it is preferred that the ligand binds to the member of the glypican family in a specific manner, that is the ligand preferentially binds to the member of the glypican family it is specific for as compared to other proteins of the cell. The specific ligands are in particular selected from the group consisting of polyclonal, monoclonal and recombinant antibodies, or fragments thereof, such as Fab fragments, F(ab')₂ fragments and scFv fragments; phage antibodies (PhAbs); llama and camel antibodies; and aptamers. Preferably, the specific ligands are monoclonal antibodies.

In the methods of purification according to the invention, the ligand of the member of the glypican family can be immobilized to facilitate the separation of stem cells from the sample. In particular, the ligand of the member of the glypican family can be immobilized via being bound to a solid support. Non-limiting examples of suitable solid supports include glass or plastic slides, resins, tissue culture plates, microtiter wells, tubes, silicon chips, or particles such as beads (including, but not limited to, latex, polystyrene, or glass beads). Any method known in the art can be used to attach the ligand of the member of the glypican family to a solid support, including use of covalent and non-covalent linkages, or passive absorption.

The invention will be further illustrated by the following figures and examples.

### Description of the figures

**Figure 1** shows the semi-quantitative RT-PCR analysis of neuronal differentiation in wild-type (wt) and *gpc4*^{mut} (mut) embryonic stem cells over 6 days. The transcription of a pluripotent marker (Oct4) and of a marker of neuronal cell lineage (nestin) is shown.
**Figure 2** shows the semi-quantitative RT-PCR analysis of cardiac differentiation in wild-type (wt) and *gpc4*^{mut} (mut) embryonic stem cells over 7 days. The transcription of a pluripotent marker (Oct4) and of a marker of cardiac cell lineage (αMHC) is shown.
**Figure 3** shows the expression of Oct4 and nestin by immuno-staining of differentiating wild-type (wt) and *gpc4*^{mut} (mut) embryonic stem cells over 6 days.
**Figure 4** shows the quantitative analysis of the expression of Oct4 and nestin in wild-type (wt) and *gpc4*^{mut} (Gpc4.mut) embryonic stem cells over 6 days. The results are shown in percentage of Oct4 and nestin positive cells. *** indicates P<0.001 and ** indicates P<0.02.
**Figure 5** shows an example of a nude mouse in which wild-type embryonic stem cells were injected in the left flank and *gpc4*^{mut} cells were injected in the right flank, 4 weeks after the injection.
**Figure 6** shows the exogenous cellular masses dissected from a mouse as shown in Figure 5.
**Figure 7** shows the weight of the exogenous cellular masses, in grams, dissected from a mouse as shown in Figure 5.
**Figure 8** shows the level of Wnt activity measured in wild-type (wt) or *gpc4*^{mut} cells when different doses of Wnt protein were added to the cells.
**Figure 9** shows the percentage of beating embryo bodies over time among wild-type cardiomyocytes and *gpc4*^{mut} cardiomyocytes.
**Figure 10** shows the expression of the dopaminergic neuronal marker (TH) by immuno-staining in differentiating wild-type (wt) or *gpc4*^{mut} cells.

### Examples

### Example 1: Cell differentiation properties of gpc4^{mut} cells

The inventors used a semi-quantitative RT-PCR strategy to assess cell differentiation properties of *gpc4*^{mut} cells by following the expression of pluripotency and lineage specific markers overtime. Immunocytochemistry was applied to quantify cell differentiation at precise time points. Finally, microscopic examination has been applied to follow neural differentiation and functional activity of developing cardiomyocytes based on the contractile activity of embryo bodies in culture plate.

### Material and methods

### Glypican 4 mutant cells

The Glypican 4 mutant cells used by the inventors were generated by W. Skarnes using a gene trap thechnology (Skarnes at al. (1995) Proc. Natl. Acad. Sci. USA 92:6592-6596). Mouse embryonic stem cells (E14Tg2a.4, derived from the 1 29/Ola mouse strain) were electroporated with the secretory trap vector (pGT1.8TM, pGT1 Mpfs). By direct sequencing of cDNAs obtained by rapid amplification of cDNA ends (RACE), ESC lines expressing a fusion transcript composed of gene trap vector sequences joined to the Glypican 4 endogenous gene were identified. The inventors confirmed that these ESC lines generated this fusion transcript by performing RT-PCR analysis using a primer in exon 1 of Glypican 4 and a second primer in the lacZ gene of the secretory trap vector. By performing genome walking they also showed that the insertion of the gene trap vector occurred in the first intron of the mouse Glypican 4 gene locus.

### Semi-quantitative RT-PCR studies

Total RNA was isolated by acid-phenol extraction (Trizol; Gibco/BRL, Gaithersburg, MD). Reverse transcription-PCR (RT-PCR) was performed with the Perkin-Elmer RT-PCR kit, as recommended by the manufacturer. cDNA was amplified by PCR. The number of cycles was chosen to select PCR conditions on the linear portion of the reaction curve to avoid the saturation effects of PCR. Sequences of specific primers, number of cycles, annealing temperature and the length of the amplified products were established for each couple of primers.

### Immunocytochemistry

Cells were fixed in 4% paraformaldehyde and 1X phosphate buffered saline (PBS) at room temperature for 30 min. Following fixation, samples were washed three times with 1X PBS for 5 min and then incubated with 10% normal goat serum and 0.1% Triton X-100 in 1X PBS for 15 min at room temperature. The cells were then washed three times in 1X PBS for 5 min and incubated with primary antibodies in 10% normal goat serum and 1X PBS at the corresponding working dilutions. Following primary antibody incubation, cells were rinsed three times in 1X PBS and further incubated with appropriate secondary antibodies in 10% normal goat serum and 1X PBS for 30 min at room temperature. Finally, samples were washed four times in 1X PBS and counterstained with 4,6-diamidino-2-phenylindole (DAPI, 250 ng/ml). Labeling was detected by fluorescent illumination using an inverted microscope.

### Results

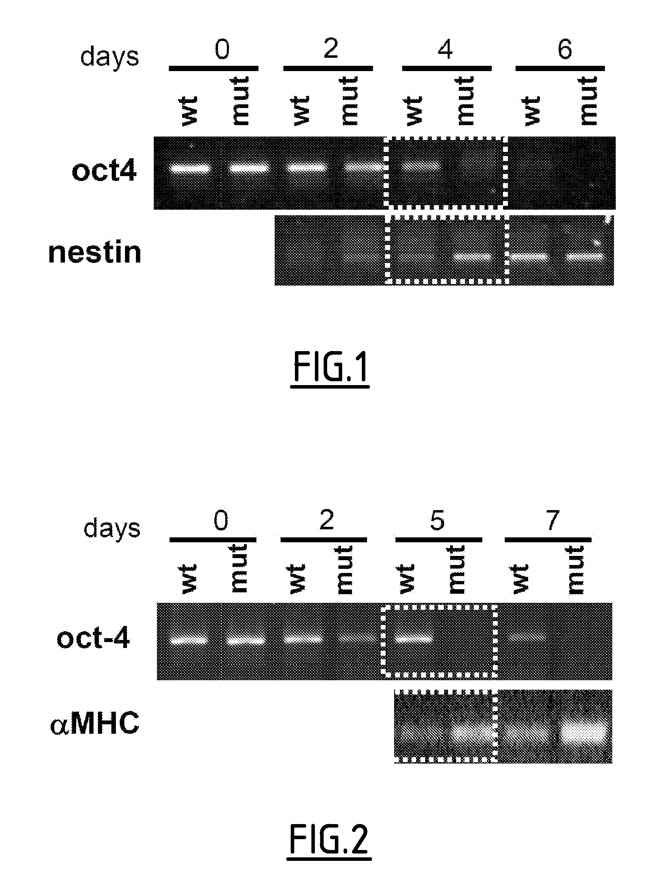

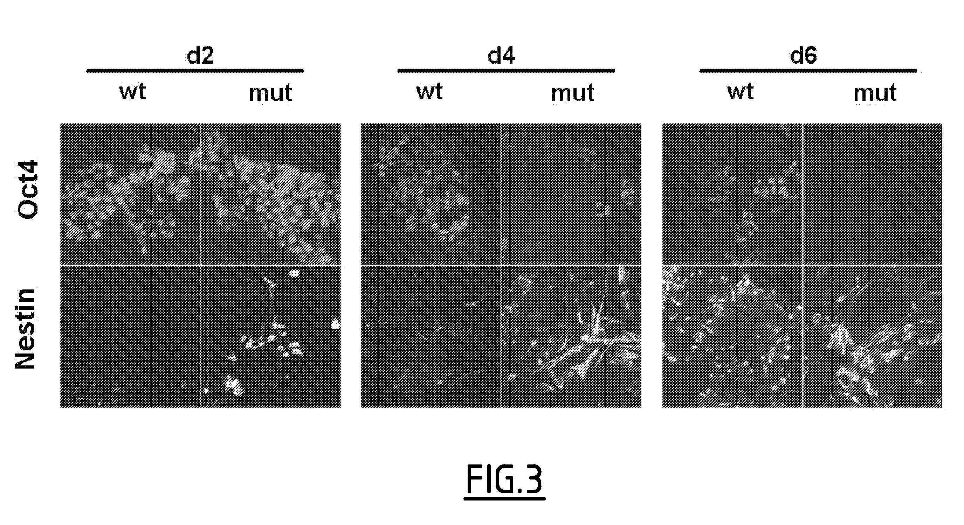

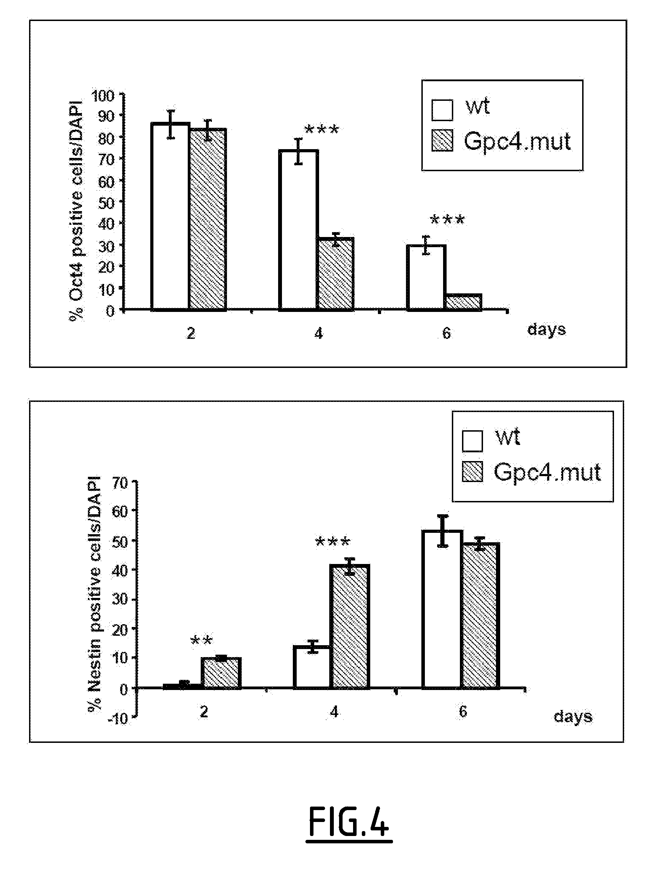

The inventors showed that *gpc4*^{mut} cells significantly down-regulated the expression of pluripotent markers such as Oct4 and up-regulated those of neuronal and cardiac lineages (nestin and αMHC respectively) 3 days earlier than wild-type embryonic stem cells (**Figures 1 and 2**). Moreover, the 60% reduction in Oct4-positive cells found in neuronal differentiating *gpc4*^{mut} cells strikingly correlated with 60% increase of nestin-positive cells (**Figures 3** **and** **4**) indicating that the missing pluripotent Oct4 cells underwent a cell fate switch in neuronal cell types.

To understand whether *gpc4*^{mut} cells retain the embryonic stem cells properties to differentiate in all cell lineages, the inventors injected *gpc4*^{mut} cells into blastocysts and followed their presence in embryo tissues. They showed that *gpc4*^{mut} cells were present in all three germ layers (ectoderm, mesoderm and endoderm). These studies proved that *gpc4*^{mut} cells retained the key property of responding to developmental signals and of differentiating in all body cell types.

### Example 2: The modulation of Glypican 4 prevents tumour formation

The results described in Example 1 drove the inventors to evaluate whether the modulation of Gpc4 would prevent tumour formation when injected *in vivo.*

Wild-type and *gpc4*^{mut} ESCs were harvested and washed with PBS and resuspended in PBS at a concentration of 5 x 10⁷ cells/ml. A total of 100 µl of cell suspension was injected subcutaneously into 6-week-old athymic nude mice (C3H/HeNCr-nu). Six mice were used for each experiment, which were done in duplicate. Mice were monitored for teratoma formation a week after the injection. The tumor development was monitored every other day for a total of four weeks. The tumors were dissected out to estalish tumour weight and to analyze them further by generating paraffin-embedded sections stained with H&E.

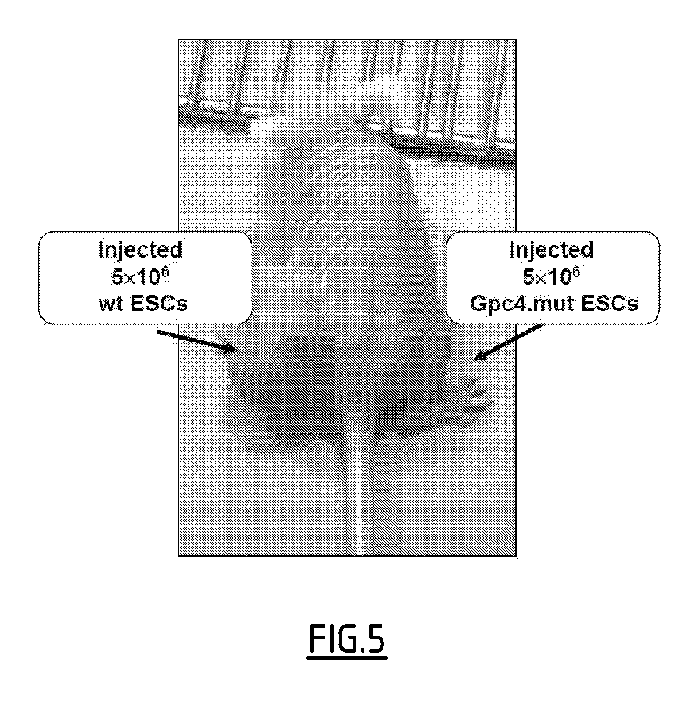

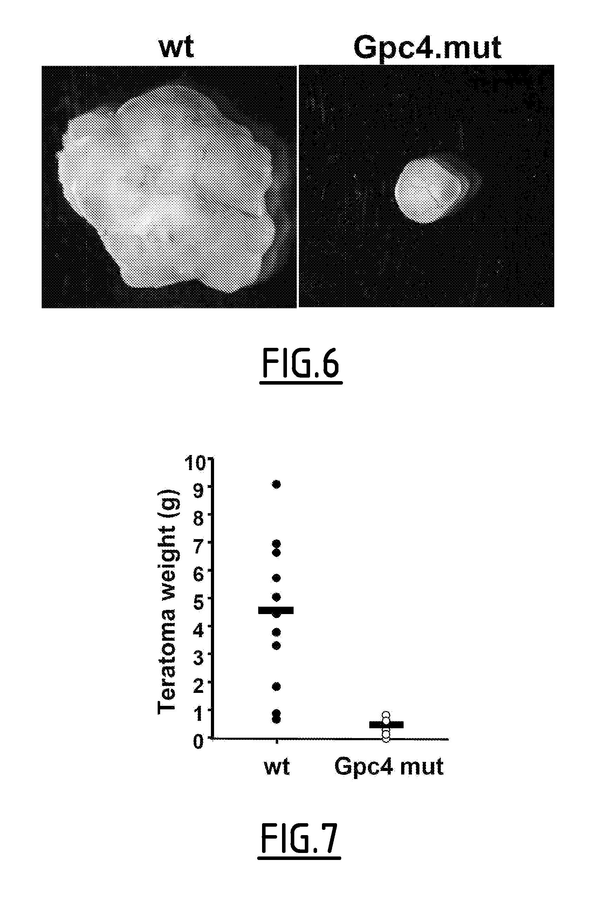

The inventors showed that when *gpc4*^{mut} cells were transplanted in the flank of nude mice, the risk of teratoma was overcome, in contrast to wild-type cells (Figure 5).Qualitative and quantitative analyses showed that *gpc4*^{mut} cells either remained as isolated cells or became part of cellular aggregates 10 times smaller than wild-type cells and did not display any teratoma morphology (**Figures 6 and 7**).

### Example 3: Molecular mechanisms underlying Gpc4 functions in stem cells

The inventors next investigated the molecular mechanisms underlying Gpc4 functions in stem cells by evaluating the possibility that Gpc4 controls environmental signals regulating cell pluripotency.

Peptide signals keeping pluripotency in embryonic stem cells include bone morphogenetic proteins, leukaemia inhibitory factor and Wnt family members.

Activation of the Wnt signalling pathway was tested by using the TOP-flash assay. Wild-type and *gpc4*^{mut} embryonic stem cells were transfected either with the TOP-FLASH luciferase reporter plasmid or its mutant control, FOP- FLASH luciferase reporter plasmid together with a Renilla luciferase-containing plasmid. The endogenous β-catenin/Tcf transcriptional complex bound to Tcf sequences upstream of the enzyme luciferase in the TOP-FLASH plasmid vector, drives luciferase expression upon Wnt pathway activation. The FOP-FLASH plasmid was used as a control because it contained mutated Tcf sequences. 24 h after transfection the cells were incubated with 5 ng, 10 ng and 50 ng/ml of Wnt3a for 24 h and lysates were collected in Reporter lysis buffer (Promega). Luciferase activity was measured in a Lumistar Galaxy luminometer (BMG Labtech) and normalized to Renilla luciferase activity. The assays were performed in duplicate in at least two independent experiments. Values were normalized to treatment with media not containing Wnt3a.

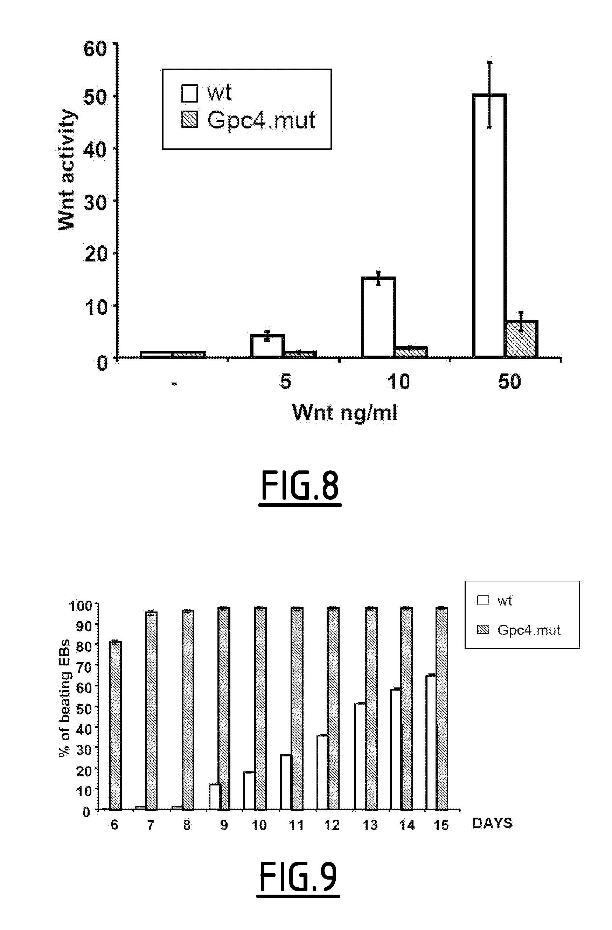

The inventors showed that Wnt activity was reduced in *gpc4*^{mut} ESCs compared to wild-type ESCs thus demonstrating that Gpc4 is needed for proper response of stem cells to Wnt signalling (**Figure 8**).

### Example 4: Use of Glypican 4 modulation for cardiac repair

The inventors showed that *gpc4*^{mut} cardiomyocytes had a more robust and persistent contractile activity than wild-type cells (**Figure 9**).

Wild-type and *gpc4*^{mut} ESCs were cultivated in EBs essentially as described in Maltsev et al. (1993) Mech Dev. 44(1):41-50. Briefly, the preparation of EBs was carried out in hanging drops (300 cells/30 µl drop) on the lid of 10 cm bacteriological dishes containing 5 ml PBS. Hanging drops containing EBs were incubated at 37°C in an atmosphere containing 5% CO₂ for two days. The hanging drops were then washed with DMEM medium supplemented with 20% FCS, re-seeded in a new bacteriological dish and incubated at 37°C for another 3 days to generate 5 days old EBs. On day 5, EBs were plated separately onto gelatin-coated 24-well plates for morphological analysis of beating cardiomyocytes or onto 100-mm tissue culture plates for RT-PCR and Western blots.

The inventors demonstrated that the percentage of the beating EBs was higher with *gpc4*^{mut} ESCs than with wild-type ESCs and that this high percentage was reached much quicker.

### Example 5: Use of Glypican 4 modulation for cell-based therapies of Parkinson disease

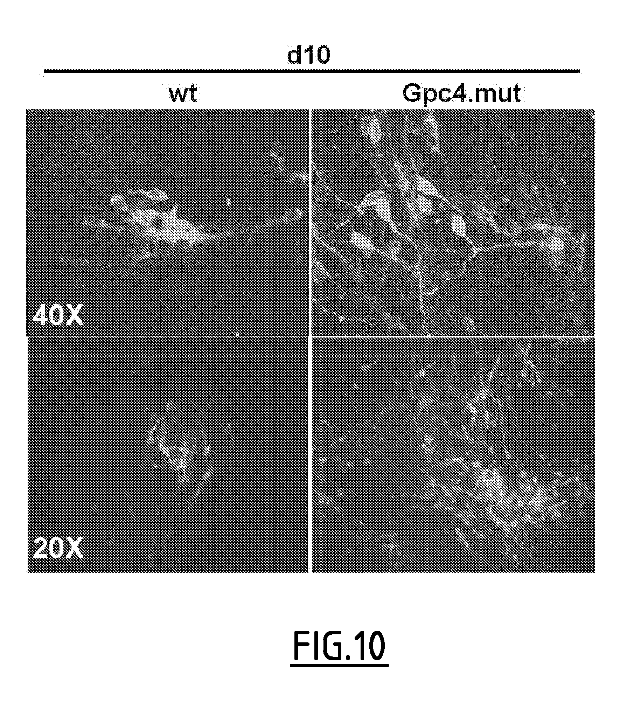

The inventors showed that *gpc4*^{mut} cells differentiated in neuronal cells had intrinsic abilities to develop high numbers of tyrosine hydroxylase neurons (15-fold more than wild-type cells), with features of midbrain dopaminergic cells even in the absence of factors, which are normally required to trigger dopaminergic cell fate (**Figure 10**).

At day 0, wild-type and *gpc4*^{mut} ESCs were dissociated in a single-cell suspension and 1,000 cells/cm² were plated on gelatin-coated plates. The culture medium was replaced daily during differentiation process. Culture medium for neuronal differentiation (serum-free Knockout Serum Replacement (KSR)-supplemented medium) contained knockout Dulbecco minimal essential medium supplemented with 15% KSR (Invitrogen), 2 mM glutamine, 100 U/ml penicillin/streptomycin, and 0.1 mM beta-mercaptoethanol. Neural differentiation was followed by morphological examination and marker analysis as described in Fico et al. (2008) Stem Cells and Development 17:573-584.

The inventors demonstrated that differentiated *gpc4*^{mut} cells developed a higher number of tyrosine hydroxylase neurons than wild-type cells.

## Claims

1. Stem cells or progenitor cells wherein the expression and/or activity of a member of the glypican family is reduced or abolished for use for the treatment by stem cell-based therapy of a pathological condition selected from the group consisting of a degenerative disease, a cardiac disorder, a metabolic disease and an injury.

2. Stem cells or progenitor cells for use according to claim 1, wherein the stem cells or progenitor cells are not directly derived from a human embryo.

3. The stem cells or progenitor cells for use according to claim 1 or 2, wherein the formation of teratomas is prevented.

4. The stem cells or progenitor cells for use according to any one of claims 1 to 3, wherein the member of the glypican family is Glypican 4.

5. An *in vitro* method for producing stem cells or progenitor cells displaying an early differentiation when exposed to environmental conditions triggering cell differentiation, comprising reducing or abolishing the expression and/or activity of a member of the glypican family in stem cells or progenitor cells;
thereby yielding stem cells or progenitor cells displaying an early differentiation when exposed to environmental conditions triggering cell differentiation.

6. The method according to claim 5, wherein the stem cells or progenitor cells are not directly derived from a human embryo.

7. An *in vitro* method for producing induced pluripotent stem cells (iPS cells) from somatic cells, comprising transiently increasing or inducing then transiently decreasing or reducing the expression and/or activity of a member of the glypican family in the somatic cells, thereby yielding induced pluripotent stem cells.

8. Stem cells, progenitor cells or iPS cells obtainable by an *in vitro* method as defined in any one of claims 5 to 7.

9. Stem cells or progenitor cells obtainable by an *in vitro* method as defined in any one of claims 5 to 6 for use for the treatment by stem cell-based therapy of a pathological condition selected from the group consisting of a degenerative disease, a cardiac disorder, a metabolic disease and an injury.

10. An *in vitro* method for producing differentiated cells from stem cells or progenitor cells comprising the steps consisting in:
a) producing stem cells or progenitor cells displaying an early differentiation when exposed to environmental conditions triggering cell differentiation by the method defined in any one of claims 5 to 6; and
b) exposing the stem cells or progenitor cells produced in step a) to environmental conditions triggering cell differentiation;
whereby differentiated cells are produced.

11. Use of stem cells, progenitor cells or iPS cells produced by the *in vitro* method as defined in any one of claims 5 to 7 for toxicological screenings.

12. Use of a member of the glypican family for specifically purifying stem cells, progenitor cells, cancer stem cells or tissue specific stem cells from a sample.

13. An *in vitro* method for specifically purifying stem cells, progenitor cells, cancer stem cells or tissue specific stem cells from a sample, wherein the sample is contacted with a ligand of a member of the glypican family;
thereby purifying stem cells, progenitor cells, cancer stem cells or tissue specific stem cells which are present in the sample.

14. A modulator of the activity of a member of the glypican family for use for the treatment of cancer.

15. An antagonist of the activity of a member of the glypican family for use for inducing tissue regeneration after a degenerative disease, preferably for enhancing differentiation of tissue specific stem cells present in the tissue to be regenerated.


SEQUENCE LISTING

<110>  CNRS
       Université de Provence-Aix Marseille II

<120>  Regulation of Glypican 4 activity to modulate the fate of stem
       cells and uses thereof

<130>  BET10P0272

<160>  9

<170>  PatentIn version 3.5

<210>  1
<211>  556
<212>  PRT
<213>  Homo sapiens


<220>
<221>  SIGNAL
<222>  (1)..(18)

<220>
<221>  SITE
<222>  (16)..(19)
<223>  Glycosaminoglycan attachment site

<220>
<221>  SITE
<222>  (358)..(361)
<223>  Glycosaminoglycan attachment site

<220>
<221>  SITE
<222>  (361)..(364)
<223>  Glycosaminoglycan attachment site

<220>
<221>  SITE
<222>  (491)..(496)
<223>  Glycosaminoglycan attachment site

<220>
<221>  SITE
<222>  (496)..(500)
<223>  Glycosaminoglycan attachment site

<220>
<221>  SITE
<222>  (521)..(524)
<223>  Glycosaminoglycan attachment site

<220>
<221>  SITE
<222>  (528)..(531)
<223>  Glycosaminoglycan attachment site

<220>
<221>  PROPEP
<222>  (530)..(556)

<400>  1

<210>  2
<211>  58

<212>  DNA
<213>  Artificial Sequence

<220>
<223>  shRNA

<400>  2
ccgggccact ggtttaagca atgttctcga gaacattgct taaaccagtg gctttttg          58


<210>  3
<211>  58
<212>  DNA
<213>  Artificial Sequence

<220>
<223>  shRNA

<400>  3
ccgggcatcc cgttacaaga agtttctcga gaaacttctt gtaacgggat gctttttg          58


<210>  4
<211>  58
<212>  DNA
<213>  Artificial Sequence

<220>
<223>  shRNA

<400>  4
ccgggcagag aaatccctga atgatctcga gatcattcag ggatttctct gctttttg          58


<210>  5
<211>  59
<212>  DNA
<213>  Artificial Sequence

<220>
<223>  shRNA

<400>  5
ccggccttca gagtttgact acaatctcga gattgtagtc aaactctgaa ggtttttttg        59


<210>  6
<211>  59
<212>  DNA
<213>  Artificial Sequence

<220>
<223>  shRNA

<400>  6
ccgggcctgc aaagtaaaga tgattctcga gaatcatctt tactttgcag gcttttttg         59


<210>  7
<211>  59
<212>  DNA
<213>  Artificial Sequence

<220>
<223>  shRNA

<400>  7
ccgggcaaat tgaagctcca ggttactcga gtaacctgga gcttcaattt gcttttttg         59

<210>  8
<211>  59
<212>  DNA
<213>  Artificial Sequence

<220>
<223>  shRNA

<400>  8
ccgggctatt atgaacatgc aggatctcga gatcctgcat gttcataata gctttttg          59


<210>  9
<211>  59
<212>  DNA
<213>  Artificial Sequence

<220>
<223>  shRNA

<400>  9
ccgggcttca cgttacaaga agtttctcga gaaacttctt gtaacgtgaa gctttttg          59